# EUROPEAN PATENT APPLICATION

(11) **EP 4 745 688 A1**
(43) Date of publication of application: **20.05.2026**
(21) Application number: 24860194.0
(22) Date of filing: 25.07.2024
(51) Int. Cl.: G04G 21/02, G06F 1/16, G04G 17/04

(54) **ELECTRONIC DEVICE COMPRISING LIGHT-EMITTING STRUCTURE**

(30) Priority: 31.08.2023 KR 20230115698; 20.10.2023 KR 20230141085
(71) Applicant: Samsung Electronics Co., Ltd., Suwon-si, Gyeonggi-do 16677 (KR)
(72) Inventor: KIM, Younghyun, Suwon-si, Gyeonggi-do 16677 (KR); AHN, Joongwoo, Suwon-si, Gyeonggi-do 16677 (KR); HWANG, Minkyung, Suwon-si, Gyeonggi-do 16677 (KR)
(74) Representative: Appleyard Lees IP LLP
(86) International application number: PCT/KR2024/010824
(87) International publication number: WO 2025/048273

(57) **Abstract**

An electronic device is provided. The electronic device includes a housing including a first surface, a second surface opposite to the first surface, and a side surface surrounding the first surface and the second surface, a substrate disposed in the housing, at least one light-emitting structure disposed on the substrate so as to be directed toward the second surface and being formed in a form of a bar, a plurality of light sources configured to emit light in different wavelength ranges and being disposed at designated intervals on the at least one light-emitting structure formed in the form of the bar, wherein the at least one light-emitting structure is electrically connected to the substrate by soldering in a state in which at least one of the at least one light-emitting structure is inclined at a particular angle.

## Description

### [Technical Field]

The disclosure relates to an electronic device including a light-emitting structure.

### [Background Art]

Recently, electronic devices in the form of wristwatches have been equipped with various sensors capable of measuring a user's bio-information. For example, an optical sensor including at least one light-emitting element and at least one light-receiving element may be included as one of various sensors. The optical sensor may measure the user's bio-information by using light in a particular wavelength range.

The above information is presented as background information only to assist with an understanding of the disclosure. No determination has been made, and no assertion is made, as to whether of any of the above might be applicable as prior art with regard to the disclosure.

### [Disclosure of Invention]

### [Technical Problem]

A required wavelength range of light may vary depending on bio-information. Because a small-scale electronic device, such as a wristwatch, has a light source that emits light only in a particular wavelength range, many light sources having various wavelength ranges may be required to measure various pieces of bio-information. However, as the number of light sources increases, a deviation in position of emitted light increases, and a position at which light reaches a biometric sample (e.g., skin) varies depending on wavelength ranges, which may cause a difference between optical paths. For example, the electronic device may be equipped with an optical focusing element to reduce the difference between the optical paths. However, because the optical focusing element has a large volume, the optical focusing element may not be suitable for the small-scale electronic device.

Aspects of the disclosure are to address at least the above-mentioned problems and/or disadvantages and to provide at least the advantages described below. Accordingly, an aspect of the disclosure is to provide an electronic device including at least one light-emitting structure including a plurality of light sources configured to emit light in different wavelength ranges.

Additional aspects will be set forth in part in the description which follows and, in part, will be apparent from the description, or may be learned by practice of the presented embodiments.

### [Solution to Problem]

In accordance with an aspect of the disclosure, an electronic device is provided. The electronic device includes a housing including a first surface, a second surface opposite to the first surface, and a side surface surrounding the first surface and the second surface, a substrate disposed in the housing, at least one light-emitting structure being disposed on the substrate so as to be directed toward the second surface and being formed in a form of a bar, and a plurality of light sources configured to emit light in different wavelength ranges and being disposed at designated intervals on the at least one light-emitting structure formed in the form of the bar, wherein the at least one light-emitting structure is electrically connected to the substrate by soldering in a state in which at least one of the at least one light-emitting structure is inclined at a particular angle.

### [Advantageous Effects of Invention]

In accordance with an aspect of the disclosure, the at least one light-emitting structure including the plurality of light sources configured to emit light in different wavelength ranges is configured at a particular angle and electrically connected to a substrate by soldering in a state in which the at least one light-emitting structure is configured at the particular angle, such that the electronic device may allow the plurality of light sources included in the at least one light-emitting structure to emit the light in different wavelength ranges at the particular angle. The plurality of light sources included in the at least one of the light-emitting structure configured at particular angles emit the light in different wavelength ranges, such that positions at which the light in respective wavelength ranges reaches a biometric sample (e.g., skin) may be similar to one another. Therefore, the electronic device may acquire various pieces of bio-information with reliability. In addition, instead of an optical focusing element having a large volume, the electronic device includes at least one light-emitting structure including the plurality of light sources configured to emit light in different wavelength ranges, which may contribute to the decrease in size of the electronic device and reduce the difficulty of the process.

Other aspects, advantages, and salient features of the disclosure will become apparent to those skilled in the art from the following detailed description, which, taken in conjunction with the annexed drawings, discloses various embodiments of the disclosure.

### [Brief Description of Drawings]

The above and other aspects, features, and advantages of certain embodiments of the disclosure will be more apparent from the following description taken in conjunction with the accompanying drawings, in which:
FIG. 1 is a block diagram of an electronic device in a network environment according to an embodiment of the disclosure;
FIG. 2 is a perspective view of a front surface of an electronic device according to an embodiment of the disclosure;
FIG. 3 is a perspective view of a rear surface of an electronic device in FIG. 2 according to an embodiment of the disclosure;
FIG. 4 is a deployed perspective view of the electronic device of FIG. 2 according to an embodiment of the disclosure;
FIG. 5 is a view for explaining at least one light-emitting structure according to an embodiment of the disclosure;
FIGS. 6A and 6B are views illustrating a state in which at least one light-emitting structure is disposed on a substrate according to various embodiments of the disclosure;
FIG. 7 is a view illustrating a state in which at least one light-emitting structure is disposed on a substrate according to an embodiment of the disclosure;
FIG. 8 is a view illustrating a state in which at least one light-emitting structure is disposed on a substrate according to an embodiment of the disclosure;
FIG. 9 is a view illustrating a state in which at least one light-emitting structure is disposed on a substrate according to an embodiment of the disclosure;
FIG. 10 is a view for explaining a method of measuring a plurality of pieces of bio-information of a user by using a plurality of light sources included in at least one light-emitting structure according to an embodiment of the disclosure;
FIG. 11 is a view for explaining a monitoring circuit according to an embodiment of the disclosure;
FIG. 12A is a view illustrating a state in which a plurality of biometric sensors is disposed according to an embodiment of the disclosure;
FIG. 12B is a view illustrating a state in which a plurality of biometric sensors is disposed according to an embodiment of the disclosure;
FIG. 13 is a view illustrating a state in which a plurality of biometric sensors is disposed according to an embodiment of the disclosure;
FIG. 14 is a view for explaining a method of measuring a plurality of pieces of bio-information by using an electronic device including at least one light-emitting structure according to an embodiment of the disclosure; and
FIG. 15 is a view for explaining a spectrum graph related to bio-information according to an embodiment of the disclosure;

The same reference numerals are used to represent the same elements throughout the drawings.

### [Mode for the Invention]

The following description with reference to the accompanying drawings is provided to assist in a comprehensive understanding of various embodiments of the disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope and spirit of the disclosure. In addition, descriptions of well-known functions and configurations may be omitted for clarity and conciseness.

The terms and words used in the following description and claims are not limited to the bibliographical meanings, but, are merely used by the inventor to enable a clear and consistent understanding of the disclosure. Accordingly, it should be apparent to those skilled in the art that the following description of various embodiments of the disclosure is provided for illustration purpose only and not for the purpose of limiting the disclosure as defined by the appended claims and their equivalents.

It is to be understood that the singular forms "a," "an," and "the" include plural referents unless the context clearly dictates otherwise. Thus, for example, reference to "a component surface" includes reference to one or more of such surfaces.

It should be appreciated that the blocks in each flowchart and combinations of the flowcharts may be performed by one or more computer programs which include instructions. The entirety of the one or more computer programs may be stored in a single memory device or the one or more computer programs may be divided with different portions stored in different multiple memory devices.

Any of the functions or operations described herein can be processed by one processor or a combination of processors. The one processor or the combination of processors is circuitry performing processing and includes circuitry like an application processor (AP, e.g. a central processing unit (CPU)), a communication processor (CP, e.g., a modem), a graphics processing unit (GPU), a neural processing unit (NPU) (e.g., an artificial intelligence (AI) chip), a Wi-Fi chip, a Bluetooth^{®} chip, a global positioning system (GPS) chip, a near field communication (NFC) chip, connectivity chips, a sensor controller, a touch controller, a finger-print sensor controller, a display drive integrated circuit (IC), an audio CODEC chip, a universal serial bus (USB) controller, a camera controller, an image processing IC, a microprocessor unit (MPU), a system on chip (SoC), an integrated circuit (IC), or the like.

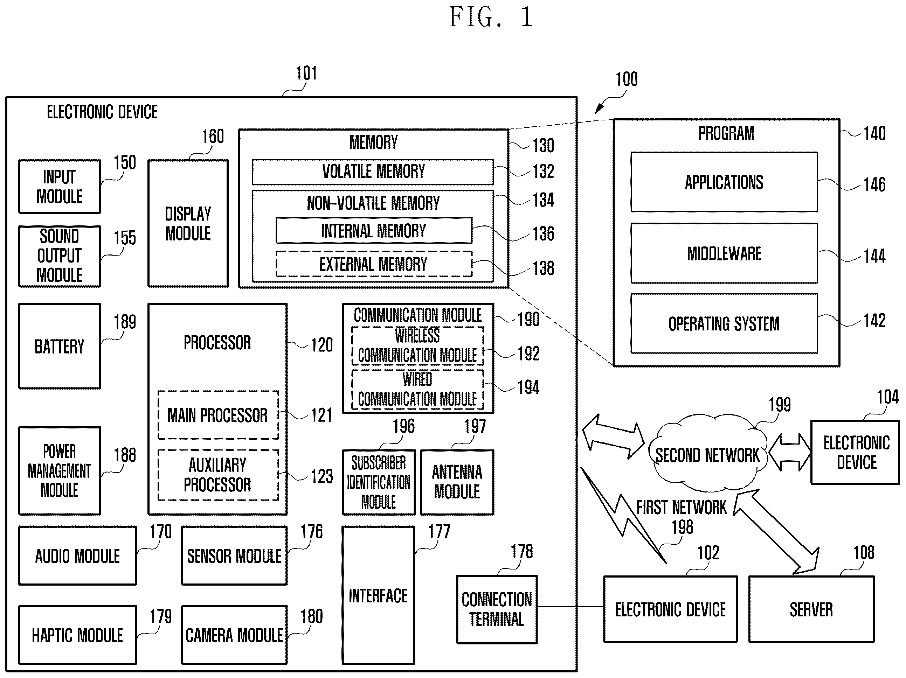

FIG. 1 is a block diagram illustrating an electronic device in a network environment according to an embodiment of the disclosure.

Referring to FIG. 1, an electronic device 101 in a network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connection terminal 178, a haptic module 179, a camera module 180, a power management module 188, a battery 189, a communication module 190, a subscriber identification module (SIM) 196, or an antenna module 197. In some embodiments, at least one of the components (e.g., the connection terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134. The non-volatile memory 134 may include internal memory 136 and/or external memory 138.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) (e.g., speaker or headphone) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., through wires) or wirelessly. According to an embodiment, the interface 177 may include, for example, a high-definition multimedia interface (HDMI), a universal serial bus (USB) interface, a secure digital (SD) card interface, or an audio interface.

The connection terminal 178 may include a connector via which the electronic device 101 may be physically connected with the external electronic device (e.g., the electronic device 102). According to an embodiment, the connection terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., an application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, Wi-Fi direct, or infrared data association (IrDA)) or the second network 199 (e.g., a long-range communication network, such as a legacy cellular network, a fifth generation (5G) network, a next-generation communication network, the Internet, or a computer network (e.g., LAN or wide area network (WAN))). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a fourth generation (4G) network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the millimeter wave (mmWave) band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large-scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20 gigabits per second (Gbps) or more) for implementing eMBB, loss coverage (e.g., 164 decibels (dB) or less) for implementing mMTC, or U-plane latency (e.g., 0.5 milliseconds (ms) or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., an mmwave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102 and 104 or the server 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

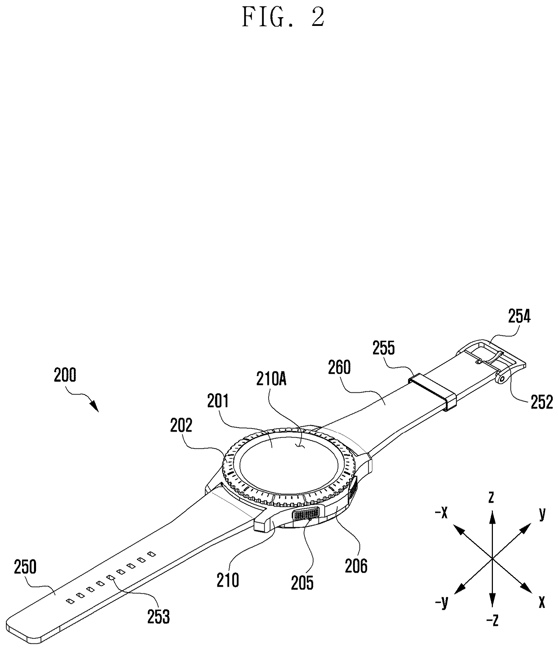

FIG. 2 is a front perspective view of an electronic device according to an embodiment of the disclosure.

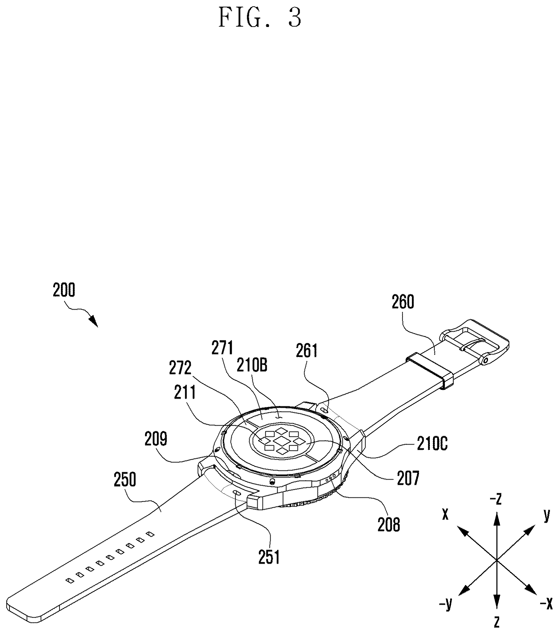

FIG. 3 is a rear perspective view of the electronic device of FIG. 2 according to an embodiment of the disclosure.

Referring to FIGS. 2 and 3, in one embodiment, an electronic device 200 (e.g., electronic device 101 in FIG 1) may include a wearable electronic device configured to detachably fasten to a body part (e.g., wrist, ankle, etc.) of the user.

In one embodiment, the electronic device 200 may include a housing 210 including a first surface (or, front surface) 210A, a second surface (or, rear surface) 210B, and a side surface 210C surrounding the space between the first surface 210A and the second surface 210B, and fastening members 250 and 260 and connected to at least a portion of the housing 210 and configured to detachably fasten the electronic device 200 to a body part (e.g., wrist, ankle, etc.) of the user. In another embodiment (not shown), the housing 210 may refer to a structure forming some of the first surface 210A, the second surface 210B, and the side surface 210C in FIG. 2. In one embodiment, the first surface 210A may be formed by a front plate 201 that is substantially transparent at least in part (e.g., glass plate containing various coating layers, or polymer plate). The second surface 210B may be formed by a rear plate 207 that is substantially opaque. The rear plate 207 may be made of, for example, coated or colored glass, ceramic, polymer, metal (e.g., aluminum, stainless steel (STS), or magnesium), or a combination thereof. The side surface 210C is coupled to the front plate 201 and the rear plate 207 and may be formed by a side bezel structure (or, side member) 206 containing metal and/or polymer. In a certain embodiment, the rear plate 207 and the side bezel structure 206 may be integrally formed and contain the same material (e.g., metal material such as aluminum). The fastening members 250 and 260 may be made of various materials and formed in various shapes. The fastening members 250 and 260 may be formed as a single body or as plural unit links that are movable with each other, by woven material, leather, rubber, urethane, metal, ceramic, or a combination thereof.

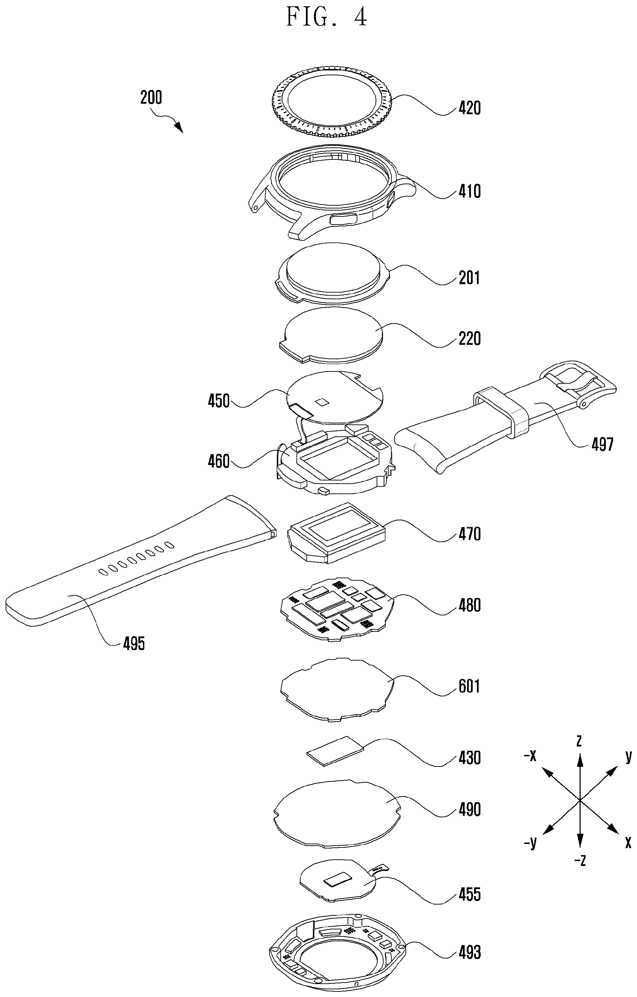

In one embodiment, the electronic device 200 may include at least one of a display (e.g., display 220 in FIG. 4), an audio module 205 and 208, a sensor module 211, key input device 202, or a connector hole 209. In a certain embodiment, at least one of the components (e.g., key input device 202, connector hole 209, or sensor module 211) may be removed from the electronic device 200, or a different component may be added to the electronic device 200.

The display 220 can be viewed through, for example, a significant portion of the front plate 201. The display 220 may have a shape corresponding to the shape of the front plate 201 and may have one of various shapes such as a circle, an ellipse, and a polygon. The display 220 may be disposed in combination with or adjacent to a touch sensing circuit, a pressure sensor capable of measuring the intensity (pressure) of a touch, and/or fingerprint sensor.

The audio module 205 and 208 may include a microphone hole 205 and a speaker hole 208. In the microphone hole 205, a microphone for picking up external sounds may be disposed therein, and plural microphones may be arranged to sense the direction of sound in a certain embodiment. The speaker hole 208 can be used for an external speaker and a call receiver. In a certain embodiment, the speaker hole 208 and the microphone hole 205 may be implemented as a single hole, or a speaker (e.g., piezo speaker) may be included without the speaker hole 208.

The sensor module 211 may generate an electrical signal or data value corresponding to an internal operating state of the electronic device 200 or an external environmental state. The sensor module 211 may include, for example, a biometric sensor module disposed on the second surface 210B of the housing 210. The electronic device 200 may further include a sensor module (not shown) including at least one of, for example, a gesture sensor, a gyro sensor, an air pressure sensor, a magnetic sensor, an acceleration sensor, a grip sensor, a color sensor, an infrared (IR) sensor, a temperature sensor, a humidity sensor, or an illuminance sensor.

In the embodiment, the sensor module 211 may include a first biometric sensor and/or a second biometric sensor 271 and 272. For example, the first biometric sensor may be a sensor including a plurality of light sources including laser diodes. The second biometric sensor 271 and 272 may be a sensor including at least one light source configured by a light-emitting diode (LED).

In the embodiment, the first biometric sensor and/or the second biometric sensor 271 and 272 may detect various pieces of bio-information of a user (e.g., saturation of percutaneous oxygen (SpO₂), heart rate (HR), photoplethysmography (PPG), electrocardiogram (ECG), galvanic skin response (GSR), electroencephalogram (EEG), and/or bioelectrical impedance analysis (BIA)).

The key input device 202 may include a wheel key 202 disposed on the first surface 210A of the housing 210 and rotatable in at least one direction. The wheel key 202 may have a shape corresponding to the shape of the front plate 201. In another embodiment, the key input device 202 may be implemented in other forms, such as soft keys, on the display 220.

The connector hole 209 may accommodate a connector (e.g., universal serial bus (USB) connector) for transmitting and receiving power and/or data to and from an external electronic device, and may include another connector hole (not shown) that can accommodate a connector for transmitting and receiving an audio signal to and from an external electronic device. The electronic device 200 may further include, for example, a connector cover (not shown) that covers at least a portion of the connector hole 209 and blocks foreign substances from entering the connector hole 209.

The fastening members 250 and 260 may be detachably fastened to at least a portion of the housing 210 by using locking members 251 and 261. The fastening members 250 and 260 may include one or more of a fixing member 252, fixing member fastening holes 253, a band guide member 254, and a band fixing ring 255.

The fixing member 252 may be configured to fix the housing 210 and the fastening members 250 and 260 to a body part (e.g., wrist, or ankle) of the user. The fixing member fastening holes 253 may fix the housing 210 and the fastening members 250 and 260 to a body part of the user in correspondence to the fixing member 252. The band guide member 254 may be configured to limit the range of movement of the fixing member 252 when the fixing member 252 engages with a fixing member fastening holes 253, so that the fastening members 250 and 260 may be fastened in close contact to a body part of the user. The band fixing ring 255 may limit the range of movement of the fastening members 250 and 260 while the fixing member 252 and the fixing member fastening hole 253 are fastened.

FIG. 4 is an exploded perspective view of the electronic device of FIG. 2 according to an embodiment of the disclosure.

Referring to FIG. 4, an electronic device 200 (e.g., electronic device 101 in FIG 1) may include a housing 410 (e.g., housing 210 in FIG. 2), a wheel key 420 (e.g., wheel key 202 in FIG. 2), a front plate 201, a display 220, a first antenna 450, a second antenna 455, a support member 460 (e.g., bracket), a battery 470, a printed circuit board 480, a substrate 601, a first biometric sensor 430, a sealing member 490, a rear plate 493, and fastening members 495, 497 (e.g., fastening members 250 and 260 in FIGS. 2 and 3). At least one of the components of the electronic device 200 may be identical or similar to at least one of the components of the electronic device 200 of FIG. 2 or FIG. 3, and repeated descriptions are omitted herein.

The support member 460 disposed inside the electronic device 200 may be formed to be connected to the housing 410 or be integrally formed with the housing 410. The support member 460 may be made of, for example, a metal material and/or a non-metal (e.g., polymer) material. The support member 460 may have one surface coupled to the display 220, or the other surface coupled to the printed circuit board 480. A processor (e.g., processor 120 in FIG. 1), memory (e.g., memory 130 in FIG. 1), and/or an interface (e.g., interface 177 in FIG. 1) may be mounted on the printed circuit board 480.

The battery 470 (e.g., battery 189 in FIG. 1) is a device for supplying power to at least one component of the electronic device 200, and may include, for example, a non-rechargeable primary cell, a rechargeable secondary cell, or a fuel cell. At least a portion of the battery 470 may be disposed substantially on the same plane as, for example, the printed circuit board 480. The battery 470 may be disposed as a single body within the electronic device 200 or may be detachably disposed from the electronic device 200.

The first antenna 450 may be disposed between the display 220 and the support member 460. The first antenna 450 may include, for example, a near field communication (NFC) antenna, a wireless charging antenna, and/or a magnetic secure transmission (MST) antenna. For example, the antenna 243 may perform short-range communication with an external device, wirelessly transmit or receive power required for charging, and transmit a short-range communication signal or a magnetic-based signal including payment data. In another embodiment, an antenna structure may be formed by using portions of the housing 410 and/or the support member 460 or a combination thereof.

The second antenna 455 may be disposed between the printed circuit board 480 and the rear plate 493. The second antenna 455 may include, for example, a near field communication (NFC) antenna, a wireless charging antenna, and/or a magnetic secure transmission (MST) antenna. For example, second antenna 455 may perform short-range communication with an external device, wirelessly transmit or receive power required for charging, and transmit a short-range communication signal or a magnetic-based signal including payment data. In another embodiment, an antenna structure may be formed by using portions of the housing 410 and/or the rear plate 493 or a combination thereof.

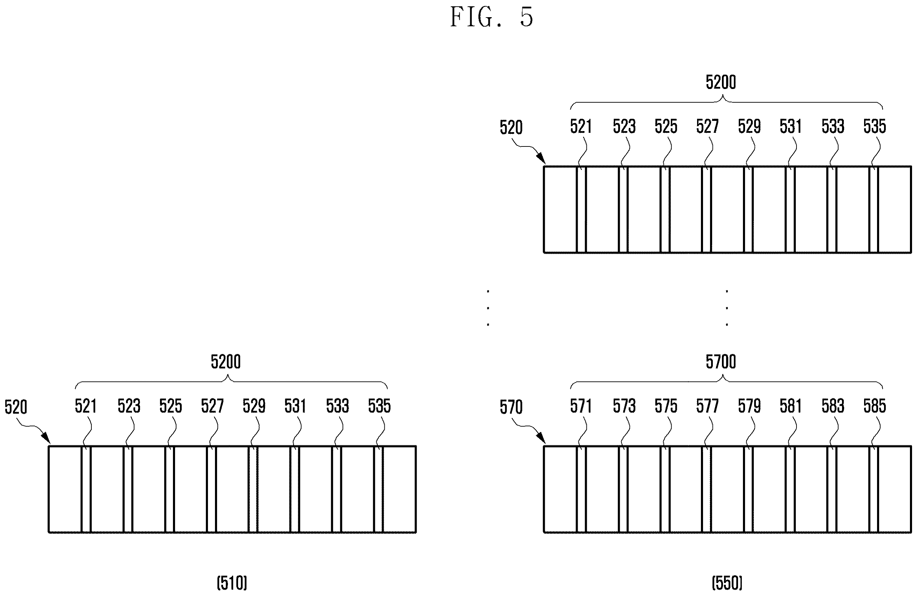

The substrate 601 may be disposed between the printed circuit board 480 and the sealing member 490. The first biometric sensor 430 may be disposed on the substrate 601. For example, the first biometric sensor 430 may include at least one light-emitting structure. The first biometric sensor 430 may be disposed on the substrate 601 so that the plurality of light sources included in each of the light-emitting structures emits light in a particular direction (e.g., the -z-axis direction). Various embodiments of the first biometric sensor 430 according to various embodiments will be described with reference to FIGS. 5, 6A, 6B, 7 to 11, 12A, 12B, and 13 to 15 to be described below.

The sealing member 490 may be positioned between the housing 410 and the rear plate 493. The sealing member 490 may be configured to block moisture and foreign matter flowing into the space surrounded by the housing 410 and the rear plate 207 from the outside.

FIG. 5 is a view for explaining at least one of light-emitting structures according to an embodiment of the disclosure.

Referring to FIG. 5, an electronic device (e.g., electronic device 101 in FIG. 1 or electronic device 200 in FIGS. 2 to 4) may include a first biometric sensor (e.g., first biometric sensor 430 in FIG. 4). The first biometric sensor 430 may include at least one of the light-emitting structures. For example, as illustrated in reference numeral <510>, the electronic device 200 may include one light-emitting structure, e.g., a first light-emitting structure 520. As another example, as illustrated in reference numeral <550>, the electronic device 200 may include a plurality of light-emitting structures, e.g., the first light-emitting structure 520, ..., and an n-th light-emitting structure 570.

Referring to reference numeral <510> according to the embodiment, the first light-emitting structure 520 may be formed in the form of a bar. In the embodiment, the first light-emitting structure 520 may include a plurality of light sources 5200. For example, the plurality of light sources 5200 may include a first-first light source 521, a first-second light source 523, a first-third light source 525, a first-fourth light source 527, a first-fifth light source 529, a first-sixth light source 531, a first-seventh light source 533, and a first-eighth light source 535.

In the embodiment, the first light-emitting structure 520 may be disposed on the substrate. In the embodiment, the substrate may include a silicon or ceramic substrate having a flat plate shape. However, the disclosure is not limited thereto.

In the embodiment, the first light-emitting structure 520 may be disposed on the substrate and directed in a particular direction (e.g., the -z-axis direction in FIG. 4). For example, the first light-emitting structure 520 may be disposed on the substrate so that the plurality of light sources 5200 included in the first light-emitting structure 520 emits light in the particular direction (e.g., the -z-axis direction in FIG. 4).

In the embodiment, the plurality of light sources 5200 may include laser diodes (LDs). The plurality of light sources 5200 may be defined as a vertical cavity surface emitting laser (VCSEL) type that emits light in the particular direction (e.g., the -z-axis direction in FIG. 4). However, the disclosure is not limited thereto.

In the embodiment, the plurality of light sources 5200 included in the first light-emitting structure 520 may be disposed at designated intervals (e.g., disposed in the form of a light source array) on the first light-emitting structure 520 formed in the form of a bar.

In the embodiment, the plurality of light sources 5200 included in the first light-emitting structure 520 may be implemented to emit light in different wavelength ranges to measure (or acquire) a plurality of pieces of bio-information. For example, the first-first light source 521 may be implemented to emit light in a first-first wavelength range (e.g., λ_{1,1}). The first-second light source 523 may be implemented to emit light in a first-second wavelength range (e.g., λ_{1,2}). The first-third light source 525 may be implemented to emit light in a first-third wavelength range (e.g., λ_{1,3}). The first-fourth light source 527 may be implemented to emit light in a first-fourth wavelength range (e.g., λ_{1,4}). The first-fifth light source 529 may be implemented to emit light in a first-fifth wavelength range (e.g., λ_{1,5}). The first-sixth light source 531 may be implemented to emit light in a first-sixth wavelength range (e.g., λ_{1,6}). The first-seventh light source 533 may be implemented to emit light in a first-seventh wavelength range (e.g., λ_{1,7}). The first-eighth light source 535 may be implemented to emit light in a first-eighth wavelength range (e.g., λ_{1,8}). The first-first wavelength range (e.g., λ_{1,1}), the first-second wavelength range (e.g., λ_{1,2}), the first-third wavelength range (e.g., λ_{1,3}), the first-fourth wavelength range (e.g., λ_{1,4}), the first-fifth wavelength range (e.g., λ_{1,5}), the first-sixth wavelength range (e.g., λ_{1,6}), the first-seventh wavelength range (e.g., λ_{1,7}), and the first-eighth wavelength range (e.g., λ_{1,8}) may be different from one another.

Referring to reference numeral <550> according to the embodiment, the plurality of light-emitting structures (e.g., the first light-emitting structure 520, ..., and the n-th light-emitting structure 570) may each include the plurality of light sources. The plurality of light sources included in the plurality of light-emitting structures (e.g., the first light-emitting structure 520, ..., and the n-th light-emitting structure 570) may be implemented to emit light in different wavelength ranges to measure the plurality of pieces of bio-information.

In the embodiment, the plurality of light-emitting structures (e.g., the first light-emitting structure 520, ..., and the n-th light-emitting structure 570) may each be formed in the form of a bar and disposed on the substrate. For example, the plurality of light-emitting structures (e.g., the first light-emitting structure 520, ..., and the n-th light-emitting structure 570) may be disposed on the substrate and directed in the particular direction (e.g., the -z-axis direction in FIG. 4). For example, the plurality of light-emitting structures (e.g., the first light-emitting structure 520, ..., and the n-th light-emitting structure 570) may be disposed on the substrate so that the plurality of light sources respectively included in the plurality of light-emitting structures (e.g., the first light-emitting structure 520, ..., and the n-th light-emitting structure 570) emits light in the particular direction (e.g., the -z-axis direction in FIG. 4).

In the embodiment, as illustrated in reference numeral <510>, the plurality of light sources 5200, which is included in the first light-emitting structure 520 among the plurality of light-emitting structures (e.g., the first light-emitting structure 520, ..., and the n-th light-emitting structure 570), may include the first-first light source 521, the first-second light source 523, the first-third light source 525, the first-fourth light source 527, the first-fifth light source 529, the first-sixth light source 531, the first-seventh light source 533, and the first-eighth light source 535. The plurality of light sources 5200 included in the first light-emitting structure 520 may be disposed at designated intervals (e.g., disposed in the form of a light source array) on the first light-emitting structure 520.

In the embodiment, the first-first light source 521 to the first-eighth light source 535, which are included in the first light-emitting structure 520, may be implemented to emit light in the first-first wavelength range to the first-eighth wavelength range. The first-first wavelength range to the first-eighth wavelength range may be different from one another.

In the embodiment, a plurality of light sources 5700, which is included in the n-th light-emitting structure 570 among the plurality of light-emitting structures (e.g., the first light-emitting structure 520, ..., and the n-th light-emitting structure 570), may include an n-th-first light source 571, an n-th-second light source 573, an n-th-third light source 575, an n-th-fourth light source 577, an n-th-fifth light source 579, an n-th-sixth light source 581, an n-th-seventh light source 583, and an n-th-eighth light source 585.

In the embodiment, the plurality of light sources 5700 included in the n-th light-emitting structure 570 may be disposed at designated intervals (e.g., disposed in the form of a light source array) on the n-th light-emitting structure 570.

In the embodiment, the plurality of light sources 5700 included in the n-th light-emitting structure 570 may be implemented to emit light in different wavelength ranges to measure (or acquire) a plurality of pieces of bio-information. For example, the n-th-first light source 571 may be implemented to emit light in an n-th-first wavelength range (e.g., λ_{n,1}). The n-th-second light source 573 may be implemented to emit light in an n-th-second wavelength range (e.g., λ_{n,2}). The n-th-third light source 575 may be implemented to emit light in an n-th-third wavelength range (e.g., λ_{n,3}). The n-th-fourth light source 577 may be implemented to emit light in an n-th-fourth wavelength range (e.g., λ_{n,4}). The n-th-fifth light source 579 may be implemented to emit light in an n-th-fifth wavelength range (e.g., λ_{n,5}). The n-th-sixth light source 581 may be implemented to emit light in an n-th-sixth wavelength range (e.g., λ_{n,6}). The n-th-seventh light source 583 may be implemented to emit light in an n-th-seventh wavelength range (e.g., λ_{n,7}). The n-th-eighth light source 585 may be implemented to emit light in an n-th-eighth wavelength range (e.g., λ_{n,8}). The n-th-first wavelength range (e.g., λ_{n,1}), the n-th-second wavelength range (e.g., λ_{n,2}), the n-th-third wavelength range (e.g., λ_{n,3}), the n-th-fourth wavelength range (e.g., λ_{n,4}), the n-th-fifth wavelength range (e.g., λ_{n,5}), the n-th-sixth wavelength range (e.g., λ_{n,6}), the n-th-seventh wavelength range (e.g., λ_{n,7}), and the n-th-eighth wavelength range (e.g., λ_{n,8}) may be different from one another.

In the embodiment, the wavelength ranges (e.g., the first-first wavelength range to the first-eighth wavelength range) of light emitted from the first-first light source 521 to the first-eighth light source 535 included in the first light-emitting structure 520 may also be different from the wavelength ranges (e.g., the n-th-first wavelength range to the n-th-eighth wavelength range) of light emitted from the n-th-first light source 571 to the n-th-eighth light source 585 included in the n-th light-emitting structure 570.

In the embodiment, the required wavelength range of light may vary depending on the bio-information. As described above, the light is emitted in different wavelength ranges (e.g., the first-first wavelength range to the first-eighth wavelength range and the n-th-first wavelength range to the n-th-eighth wavelength range) from the first-first light source 521 to the first-eighth light source 535, which are included in the first light-emitting structure 520, and the n-th-first light source 571 to the n-th-eighth light source 585, which are included in the n-th light-emitting structure 570, such that it is possible to measure (or acquire) various pieces of bio-information on the basis of the light in the different wavelength ranges.

In reference numerals <510> and <550> in FIG. 5 according to the embodiment, the configuration has been described in which the plurality of light-emitting structures (e.g., the first light-emitting structure 520, ..., and the n-th light-emitting structure 570) each includes the eight light sources. However, the disclosure is not limited thereto. For example, the plurality of light-emitting structures may each include less or more than eight light sources.

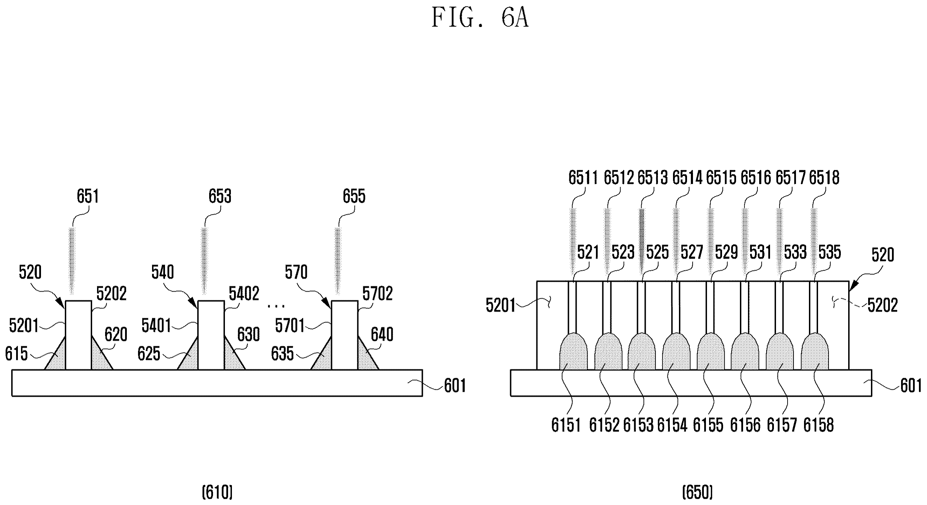

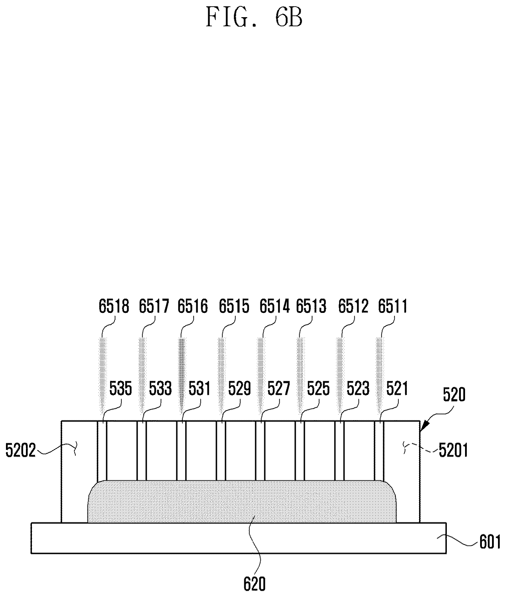

FIGS. 6A and 6B are views illustrating a state in which at least one of light-emitting structures is disposed on a substrate according to various embodiments of the disclosure.

Referring to FIGS. 6A and 6B, an electronic device (e.g., electronic device 101 in FIG. 1 or electronic device 200 in FIGS. 2 to 4) may include a first biometric sensor (e.g., first biometric sensor 430 in FIG. 4). The first biometric sensor 430 may include at least one of the light-emitting structures.

Referring to reference numeral <610> according to the embodiment, the electronic device 200 may include the first light-emitting structure 520, a second light-emitting structure 540, ..., and the n-th light-emitting structure 570. The first light-emitting structure 520, the second light-emitting structure 540, ..., and the n-th light-emitting structure 570 may each be formed in the form of a bar.

In the embodiment, the first light-emitting structure 520, the second light-emitting structure 540, ..., and the n-th light-emitting structure 570 may be disposed on the substrate 601. For example, the first light-emitting structure 520, the second light-emitting structure 540, ..., and the n-th light-emitting structure 570 may be disposed on the substrate 601 so that the plurality of light sources included in each of the light-emitting structures emits light in the particular direction (e.g., the -z-axis direction in FIG. 4).

In the embodiment, the substrate 601 may include a silicon or ceramic substrate having a flat plate shape. However, the disclosure is not limited thereto.

In the embodiment, the substrate 601, on which the first light-emitting structure 520, the second light-emitting structure 540, ..., and the n-th light-emitting structure 570 (e.g., the first biometric sensor 430) are disposed, may be disposed between the printed circuit board (PCB) 480 and the sealing member 490 in FIG. 4. However, the disclosure is not limited thereto. The substrate 601 may be the printed circuit board 480 illustrated in FIG. 4. In this case, the first light-emitting structure 520, the second light-emitting structure 540, ..., and the n-th light-emitting structure 570 may be disposed on one surface (e.g., a surface directed toward the -z-axis) of the printed circuit board 480.

In the embodiment, the first light-emitting structure 520, the second light-emitting structure 540, ..., and the n-th light-emitting structure 570 may each include the plurality of light sources, and the plurality of light sources may be implemented to emit light in different wavelength ranges to measure the plurality of pieces of bio-information.

In the embodiment, the first light-emitting structure 520 may include the plurality of light sources implemented to emit light 651 in the first-first wavelength range (e.g., λ_{1,1}) to the first-eighth wavelength range (e.g., λ_{1,8}). For example, as illustrated in reference numeral <650> in FIGS. 6A and 6B, the first light-emitting structure 520 may include the first-first light source 521 implemented to emit light 6511 in the first-first wavelength range (e.g., λ_{1,1}), the first-second light source 523 implemented to emit light 6512 in the first-second wavelength range (e.g., λ_{1,2}), the first-third light source 525 implemented to emit light 6513 in the first-third wavelength range (e.g., λ_{1,3}), the first-fourth light source 527 implemented to emit light 6514 in the first-fourth wavelength range (e.g., λ_{1,4}), the first-fifth light source 529 implemented to emit light 6515 in the first-fifth wavelength range (e.g., λ_{1,5}), the first-sixth light source 531 implemented to emit light 6516 in the first-sixth wavelength range (e.g., λ_{1,6}), the first-seventh light source 533 implemented to emit light 6517 in the first-seventh wavelength range (e.g., λ_{1,7}), and the first-eighth light source 535 implemented to emit light 6518 in the first-eighth wavelength range (e.g., λ_{1,8}).

Although not illustrated, the second light-emitting structure 540 may include a plurality of light sources implemented to emit light 653 in a second-first wavelength range (e.g., λ_{2,1}) to a second-eighth wavelength range (e.g., λ_{2,8}). The n-th light-emitting structure 570 may include the plurality of light sources implemented to emit light 655 in the n-th-first wavelength range (e.g., λ_{n,1}) to the n-th-eighth wavelength range (e.g., λ_{n,8}).

In the embodiment, the first-first wavelength range (e.g., λ_{1,1}) to the first-eighth wavelength range (e.g., λ_{1,8}), the second-first wavelength range (e.g., λ_{2,1}) to the second-eighth wavelength range (e.g., λ_{2,8}), and the n-th-first wavelength range (e.g., λ_{n,1}) to the n-th-eighth wavelength range (e.g., λ_{n,8}) may be different from one another.

In the embodiment, the plurality of light sources included in the first light-emitting structure 520 may be disposed at designated intervals (e.g., disposed in the form of a light source array) on the first light-emitting structure 520 formed in the form of a bar.

Although not illustrated, the plurality of light sources included in each of the other light-emitting structures (e.g., the second light-emitting structure 540, ..., and the n-th light-emitting structure 570) may also be disposed at designated intervals (e.g., disposed in the form of a light source array) on the corresponding light-emitting structures (e.g., the second light-emitting structure 540, ..., and the n-th light-emitting structure 570) formed in the form of a bar.

In the embodiment, the first light-emitting structure 520, the second light-emitting structure 540, ..., and the n-th light-emitting structure 570 may be electrically connected to the substrate 601 by means of an electrical connection structure such as soldering. For example, the first light-emitting structure 520 may be electrically connected to the substrate 601 by means of a first soldering member 615, which is disposed on at least a part of a first surface 5201 of the first light-emitting structure 520, and a second soldering member 620 disposed on at least a part of a second surface 5202 of the first light-emitting structure 520. For example, the second light-emitting structure 540 may be electrically connected to the substrate 601 by means of a third soldering member 625, which is disposed on at least a part of a first surface 5401 of the second light-emitting structure 540, and a fourth soldering member 630 disposed on at least a part of a second surface 5402 of the second light-emitting structure 540. For example, the n-th light-emitting structure 570 may be electrically connected to the substrate 601 by means of a fifth soldering member 635, which is disposed on at least a part of a first surface 5701 of the n-th light-emitting structure 570, and a sixth soldering member 640 disposed on at least a part of a second surface 5702 of the n-th light-emitting structure 570.

In the embodiment, the soldering members (e.g., the first soldering member 615, the second soldering member 620, the third soldering member 625, the fourth soldering member 630, the fifth soldering member 635, and the sixth soldering member 640) may be made of at least one of gold, silver, copper, tin, indium, and silicon having conductivity.

Reference numeral <650> in FIG. 6A according to various embodiments is a view when the substrate 601 and the first light-emitting structure 520 are viewed in one direction. As described above, the first light-emitting structure 520 may be electrically connected to the substrate 601 by means of the first soldering member 615 disposed on at least a part of the first surface 5201 of the first light-emitting structure 520. For example, the first soldering member 615 may include a plurality of first soldering members. The plurality of first soldering members may include a first-first soldering member 6151, a first-second soldering member 6152, a first-third soldering member 6153, a first-fourth soldering member 6154, a first-fifth soldering member 6155, a first-sixth soldering member 6156, a first-seventh soldering member 6157, and a first-eighth soldering member 6158.

For example, the first-first soldering member 6151 may be disposed on the first surface 5201 of the first light-emitting structure 520 so as to overlap at least a part of the first-first light source 521 and electrically connect the first light-emitting structure 520 and the substrate 601. The first-second soldering member 6152 may be disposed on the first surface 5201 of the first light-emitting structure 520 so as to overlap at least a part of the first-second light source 523 and electrically connect the first light-emitting structure 520 and the substrate 601. The first-third soldering member 6153 may be disposed on the first surface 5201 of the first light-emitting structure 520 so as to overlap at least a part of the first-third light source 525 and electrically connect the first light-emitting structure 520 and the substrate 601. The first-fourth soldering member 6154 may be disposed on the first surface 5201 of the first light-emitting structure 520 so as to overlap at least a part of the first-fourth light source 527 and electrically connect the first light-emitting structure 520 and the substrate 601. The first-fifth soldering member 6155 may be disposed on the first surface 5201 of the first light-emitting structure 520 so as to overlap at least a part of the first-fifth light source 529 and electrically connect the first light-emitting structure 520 and the substrate 601. The first-sixth soldering member 6156 may be disposed on the first surface 5201 of the first light-emitting structure 520 so as to overlap at least a part of the first-sixth light source 531 and electrically connect the first light-emitting structure 520 and the substrate 601. The first-seventh soldering member 6157 may be disposed on the first surface 5201 of the first light-emitting structure 520 so as to overlap at least a part of the first-seventh light source 533 and electrically connect the first light-emitting structure 520 and the substrate 601. The first-eighth soldering member 6158 may be disposed on the first surface 5201 of the first light-emitting structure 520 so as to overlap at least a part of the first-eighth light source 535 and electrically connect the first light-emitting structure 520 and the substrate 601.

FIG. 6B according to various embodiments is a view when the substrate 601 and the first light-emitting structure 520 are viewed in the other direction (e.g., a direction opposite to one direction in reference numeral <650>). As described above, the first light-emitting structure 520 may be electrically connected to the substrate 601 by means of the second soldering member 620 disposed on at least a part of the second surface 5202 of the first light-emitting structure 520. For example, the first light-emitting structure 520 may be electrically connected to the substrate 601 by means of the second soldering member 620 disposed on the second surface 5202 of the first light-emitting structure 520 so as to overlap at least a part of each of the first-first light source 521, the first-second light source 523, the first-third light source 525, the first-fourth light source 527, the first-fifth light source 529, the first-sixth light source 531, the first-seventh light source 533, and the first-eighth light source 535.

However, the disclosure is not limited thereto. Although not illustrated, like the first soldering member 615 described above with reference to reference numeral <650> in FIG. 6A, the second soldering member 620 may also include a plurality of second soldering members. In this case, the plurality of second soldering members may each be disposed on the second surface 5202 of the first light-emitting structure 520 so as to overlap each of the light sources (e.g., the first-first light source 521, the first-second light source 523, the first-third light source 525, the first-fourth light source 527, the first-fifth light source 529, the first-sixth light source 531, the first-seventh light source 533, and the first-eighth light source 535).

Although not illustrated, another light-emitting structure, for example, the second light-emitting structure 540, may be electrically connected to the substrate 601 by means of the third soldering member 625 and the fourth soldering member 630 with substantially the same shape (or structure) as the first light-emitting structure 520 described with reference to reference numeral <650> and FIG. 6B. In addition, although not illustrated, another light-emitting structure, for example, the n-th light-emitting structure 570 may be electrically connected to the substrate 601 by means of the fifth soldering member 635 and the sixth soldering member 640 with substantially the same shape (or structure) as the first light-emitting structure 520 described with reference to reference numeral <650> in FIGS. 6A and 6B.

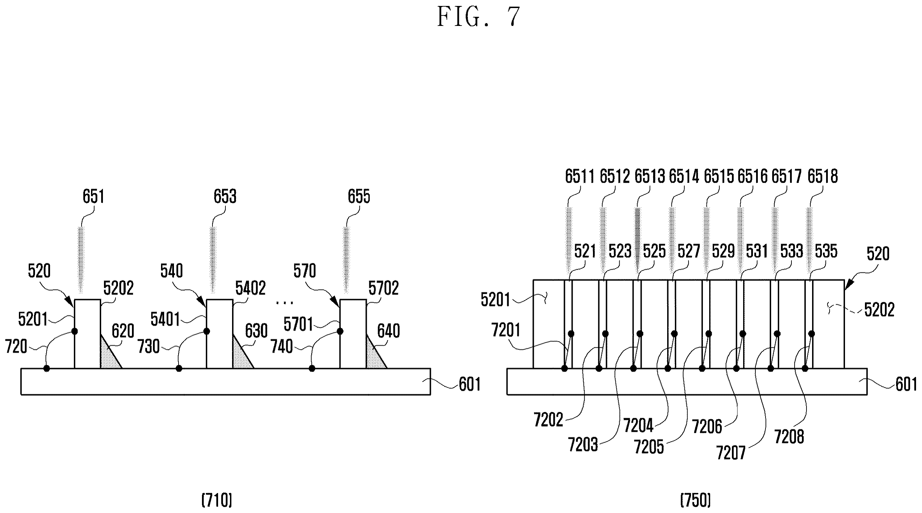

Referring to FIGS. 6A and 6B, the configuration has been described in which the first light-emitting structure 520 is electrically connected to the substrate 601 by means of the first soldering member 615 and the second soldering member 620, the second light-emitting structure 540 is electrically connected to the substrate 601 by means of the third soldering member 625 and the fourth soldering member 630, and the n-th light-emitting structure 570 is electrically connected to the substrate 601 by means of the fifth soldering member 635 and the sixth soldering member 640. However, the disclosure is not limited thereto. For example, the first light-emitting structure 520, the second light-emitting structure 540, ..., and the n-th light-emitting structure 570 may be electrically connected to the substrate 601 by means of wires instead of the first soldering member 615, the third soldering member 625, and the fifth soldering member 635. This configuration will be described in detail with reference to FIG. 7 to be described below.

FIG. 7 is a view illustrating a state in which at least one of light-emitting structures is disposed on a substrate according to an embodiment of the disclosure.

FIG. 7 according to various embodiments is a view for explaining a structure in which a first light-emitting structure 520, a second light-emitting structure 540, ..., and an n-th light-emitting structure 570, which have been described above with reference to FIGS. 6A and 6B, are electrically connected to a substrate 601 through wires instead of a first soldering member 615 disposed on at least a part of a first surface 5201 of the first light-emitting structure 520, a third soldering member 625 disposed on at least a part of a first surface 5401 of the second light-emitting structure 540, and a fifth soldering member 635 disposed on at least a part of a first surface 5701 of the n-th light-emitting structure 570.

In the following description with reference to FIG. 7, only the configuration different from the configuration described with reference to FIGS. 6A and 6B will be described.

Referring to reference numeral <710> in FIG. 7 according to the embodiment, the electronic device (e.g., electronic device 101 in FIG. 1 or electronic device 200 in FIGS. 2 to 4) may include the first light-emitting structure 520, the second light-emitting structure 540, ..., and the n-th light-emitting structure 570.

In the embodiment, the first light-emitting structure 520, the second light-emitting structure 540, ..., and the n-th light-emitting structure 570 may be electrically connected to the substrate 601 by means of the electrical connection structure such as wire and/or soldering members. For example, the first light-emitting structure 520 may be electrically connected to the substrate 601 by means of a first wire 720, which is disposed on at least a part of the first surface 5201 of the first light-emitting structure 520, and the second soldering member 620 disposed on at least a part of the second surface 5202 of the first light-emitting structure 520. For example, the second light-emitting structure 540 may be electrically connected to the substrate 601 by means of a second wire 730, which is disposed on at least a part of the first surface 5401 of the second light-emitting structure 540, and the fourth soldering member 630 disposed on at least a part of the second surface 5402 of the second light-emitting structure 540. For example, the n-th light-emitting structure 570 may be electrically connected to the substrate 601 by means of a third wire 740, which is disposed on at least a part of the first surface 5701 of the n-th light-emitting structure 570, and the sixth soldering member 640 disposed on at least a part of the second surface 5702 of the n-th light-emitting structure 570.

Reference numeral <750> in FIG. 7 according to various embodiments is a view when the substrate 601 and the first light-emitting structure 520 are viewed in one direction. As described above, the first light-emitting structure 520 may be electrically connected to the substrate 601 by means of the first wire 720 disposed on at least a part of the first surface 5201 of the first light-emitting structure 520. For example, the first wire 720 may include a plurality of first wires. The plurality of first wires may include a first-first wire 7201, a first-second wire 7202, a first-third wire 7203, a first-fourth wire 7204, a first-fifth wire 7205, a first-sixth wire 7206, a first-seventh wire 7207, and a first-eighth wire 7208.

For example, the first-first wire 7201 may be connected to at least a part of the first-first light source 521 on the first surface 5201 of the first light-emitting structure 520 and electrically connect the first light-emitting structure 520 and the substrate 601. The first-second wire 7202 may be connected to at least a part of the first-second light source 523 on the first surface 5201 of the first light-emitting structure 520 and electrically connect the first light-emitting structure 520 and the substrate 601. The first-third wire 7203 may be connected to at least a part of the first-third light source 525 on the first surface 5201 of the first light-emitting structure 520 and electrically connect the first light-emitting structure 520 and the substrate 601. The first-fourth wire 7204 may be connected to at least a part of the first-fourth light source 527 on the first surface 5201 of the first light-emitting structure 520 and electrically connect the first light-emitting structure 520 and the substrate 601. The first-fifth wire 7205 may be connected to at least a part of the first-fifth light source 529 on the first surface 5201 of the first light-emitting structure 520 and electrically connect the first light-emitting structure 520 and the substrate 601. The first-sixth wire 7206 may be connected to at least a part of the first-sixth light source 531 on the first surface 5201 of the first light-emitting structure 520 and electrically connect the first light-emitting structure 520 and the substrate 601. The first-seventh wire 7207 may be connected to at least a part of the first-seventh light source 533 on the first surface 5201 of the first light-emitting structure 520 and electrically connect the first light-emitting structure 520 and the substrate 601. The first-eighth wire 7208 may be connected to at least a part of the first-eighth light source 535 on the first surface 5201 of the first light-emitting structure 520 and electrically connect the first light-emitting structure 520 and the substrate 601.

In the embodiment, as described above with reference to FIG. 6B, the first light-emitting structure 520 may be electrically connected to the substrate 601 by means of the second soldering member 620 disposed on at least a part of the second surface 5202 of the first light-emitting structure 520.

Although not illustrated, another light-emitting structure, for example, the second light-emitting structure 540, may be electrically connected to the substrate 601 by means of the second wire 730 (e.g., a plurality of second wires) and the fourth soldering member 630 with substantially the same shape (or structure) as the first light-emitting structure 520 described above with reference to reference numeral <750> in FIGS. 7 and 6B. In addition, although not illustrated, another light-emitting structure, for example, the n-th light-emitting structure 570, may be electrically connected to the substrate 601 by means of the third wire 740 (e.g., a plurality of third wires) and the sixth soldering member 640 with substantially the same shape (or structure) as the first light-emitting structure 520 described above with reference to reference numeral <750> in FIGS. 7 and 6B.

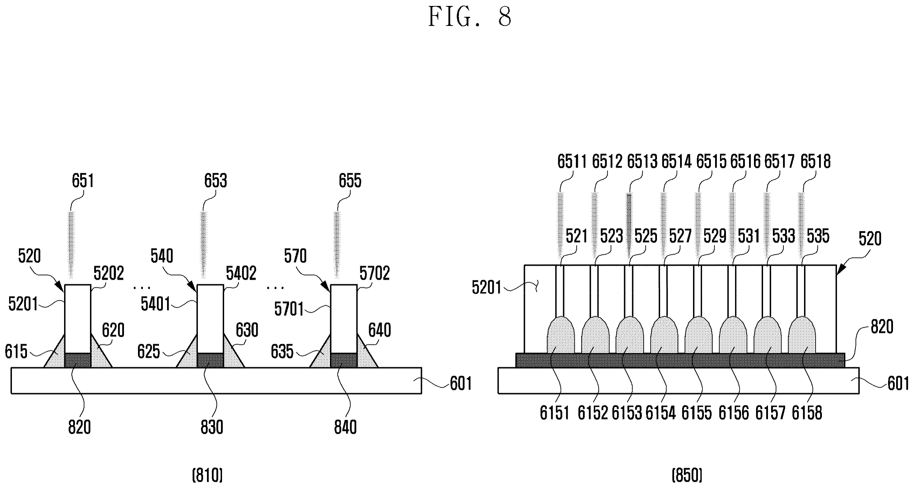

FIG. 8 is a view illustrating a state in which at least one of light-emitting structures is disposed on a substrate according to an embodiment of the disclosure.

FIG. 8 according to various embodiments is a view for explaining a structure in which at least one light absorption structure (e.g., a first light absorption structure, a second light absorption structure, ..., and an n-th light absorption structure) is additionally disposed on a substrate in the structure illustrated in FIGS. 6A and 6B described above.

In the following description with reference to FIG. 8, only the configuration different from the configuration described with reference to FIGS. 6A and 6B will be described.

Reference numeral <810> in FIG. 8 according to the embodiment is a view illustrating a state in which at least one of light absorption structures and a plurality of light-emitting structures are disposed on substrate. Reference numeral <850> in FIG. 8 is a view when a substrate, a first light absorption structure, and a first light-emitting structure 520 are viewed in one direction.

Referring to reference numerals <810> and <850> in FIG. 8 according to the embodiment, an electronic device (e.g., electronic device 101 in FIG. 1 or electronic device 200 in FIGS. 2 to 4) may include at least one of the light absorption structures disposed on a substrate 601. For example, a first light absorption structure 820, a second light absorption structure 830, ..., and an n-th light absorption structure 840 may be disposed on the substrate 601.

In the embodiment, the first light-emitting structure 520, the second light-emitting structure 540, ..., and the n-th light-emitting structure 570 may be disposed above the first light absorption structure 820, the second light absorption structure 830, ..., and the n-th light absorption structure 840. For example, in the state in which the first light-emitting structure 520, the second light-emitting structure 540, ..., and the n-th light-emitting structure 570 are disposed above the first light absorption structure 820, the second light absorption structure 830, ..., and the n-th light absorption structure 840, the first light-emitting structure 520, the second light-emitting structure 540, ..., and the n-th light-emitting structure 570 may be electrically connected to the substrate 601 by means of the electrical connection structure such as the soldering members 615, 620, 625, 630, 635, and 640.

In the embodiment, at least a part of the light emitted from the plurality of light sources, which is included in each of the light-emitting structures (e.g., the first light-emitting structure 520, the second light-emitting structure 540, ..., and the n-th light-emitting structure 570), may be transmitted to the substrate 601. In order to prevent at least a part of the light transmitted to the substrate 601 from being reflected from the substrate 601 and propagating in the particular direction (e.g., the -z-axis direction in FIG. 4), the electronic device 200 may include at least one of the light absorption structures (e.g., the first light absorption structure 820, the second light absorption structure 830, ..., and the n-th light absorption structure 840) for absorbing at least a part of the light transmitted to the substrate 601.

For example, when the plurality of light sources included in the first light-emitting structure 520 sequentially emits light in the particular direction (e.g., the -z-axis direction in FIG. 4), at least a part of the emitted light may be transmitted to the substrate 601, and the first light absorption structure 820 may absorb at least a part of the light transmitted to the substrate 601. For example, when the plurality of light sources included in the second light-emitting structure 540 sequentially emits light in the particular direction (e.g., the -z-axis direction in FIG. 4), at least a part of the emitted light may be transmitted to the substrate 601, and the second light absorption structure 830 may absorb at least a part of the light transmitted to the substrate 601. For example, when the plurality of light sources included in the n-th light-emitting structure 570 sequentially emits light in the particular direction (e.g., the -z-axis direction in FIG. 4), at least a part of the emitted light may be transmitted to the substrate 601, and the n-th light absorption structure 840 may absorb at least a part of the light transmitted to the substrate 601.

Referring to FIG. 8, the configuration has been described in which at least one of the light absorption structures (e.g., the first light absorption structure 820, the second light absorption structure 830, ..., and the n-th light absorption structure 840) is additionally included in the structure illustrated in FIGS. 6A and 6B described above. However, the disclosure is not limited thereto. For example, at least one of the light absorption structures (e.g., the first light absorption structure 820, the second light absorption structure 830, ..., and the n-th light absorption structure 840) may be additionally included in the structure illustrated in FIG. 7 described above. In this case, the first light-emitting structure 520, the second light-emitting structure 540, ..., and the n-th light-emitting structure 570 may be electrically connected to the substrate 601 by means of the electrical connection structure such as the wires 720, 730, and 740 and/or the soldering members 620, 630, and 640 in a state in which the first light-emitting structure 520, the second light-emitting structure 540, ..., and the n-th light-emitting structure 570 are disposed above the first light absorption structure 820, the second light absorption structure 830, ..., and the n-th light absorption structure 840.

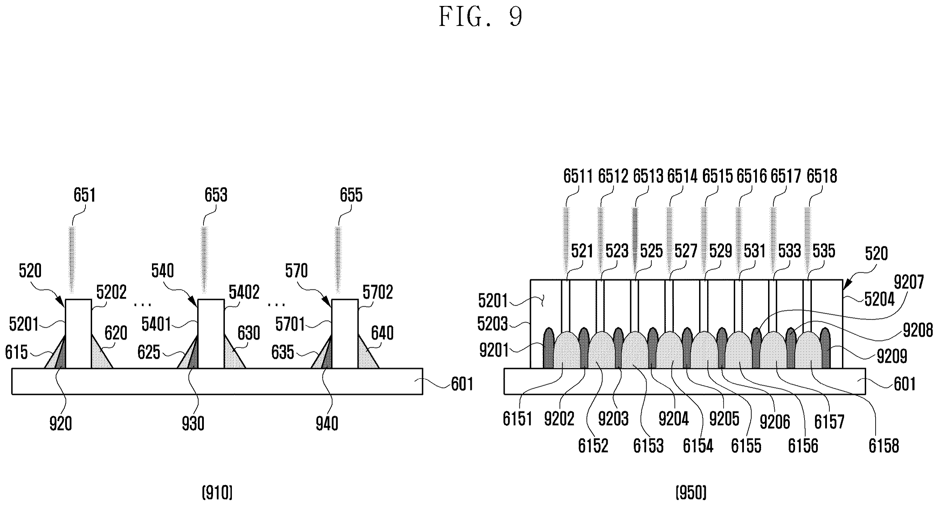

FIG. 9 is a view illustrating a state in which at least one of light-emitting structures is disposed on a substrate according to an embodiment of the disclosure.

FIG. 9 according to various embodiments is a view for explaining a structure in which at least one insulator is additionally disposed in the structure illustrated in FIGS. 6A and 6B described above.

In the following description with reference to FIG. 9, only the configuration different from the configuration described with reference to FIGS. 6A and 6B will be described.

Referring to reference numeral <910> in FIG. 9 according to the embodiment, an electronic device (e.g., electronic device 101 in FIG. 1 or electronic device 200 in FIGS. 2 to 4) may include at least one insulator disposed on at least a part of the first surface (e.g., first surface 5201 of first light-emitting structure 520, first surface 5401 of second light-emitting structure 540, ..., and first surface 5701 of n-th light-emitting structure 570) of each of the light-emitting structures (e.g., the first light-emitting structure 520, the second light-emitting structure 540, ..., and the n-th light-emitting structure 570).

For example, the electronic device 200 may include a first insulator 920 disposed on at least a part of the first surface 5201 of the first light-emitting structure 520, a second insulator 930 disposed on at least a part of the first surface 5401 of the second light-emitting structure 540, ..., and an n-th insulator 940 disposed on at least a part of the first surface 5701 of the n-th light-emitting structure 570.

Reference numeral <950> in FIG. 9 according to the embodiment is a view when the substrate 601, at least one of the insulators, and the first light-emitting structure 520 are viewed in one direction. The first insulator 920, which is disposed on at least a part of the first surface 5201 of the first light-emitting structure 520 may include a plurality of first insulators. The plurality of first insulators may include a first-first insulator 9201, a first-second insulator 9202, a first-third insulator 9203, a first-fourth insulator 9204, a first-fifth insulator 9205, a first-sixth insulator 9206, a first-seventh insulator 9207, a first-eighth insulator 9208, and a first-ninth insulator 9209.

In the embodiment, the plurality of first insulators may be respectively disposed between the plurality of light sources to insulate the plurality of light sources included in the first light-emitting structure 520. For example, the first-first insulator 9201 may be disposed between a first side surface 5203 of the first light-emitting structure 520 and the first-first light source 521. The first-second insulator 9202 may be disposed between the first-first light source 521 and the first-second light source 523. The first-third insulator 9203 may be disposed between the first-second light source 523 and the first-third light source 525. The first-fourth insulator 9204 may be disposed between the first-third light source 525 and the first-fourth light source 527. The first-fifth insulator 9205 may be disposed between the first-fourth light source 527 and the first-fifth light source 529. The first-sixth insulator 9206 may be disposed between the first-fifth light source 529 and the first-sixth light source 531. The first-seventh insulator 9207 may be disposed between the first-sixth light source 531 and the first-seventh light source 533. The first-eighth insulator 9208 may be disposed between the first-seventh light source 533 and the first-eighth light source 535. The first-ninth insulator 9209 may be disposed between the first-eighth light source 535 and a second side surface 5204 of the first light-emitting structure 520.

In the embodiment, the first insulator 920 may be disposed on at least a part of the first surface 5201 of the first light-emitting structure 520, the second insulator 930 may be disposed on at least a part of the first surface 5401 of the second light-emitting structure 540, the n-th insulator 940 may be disposed on at least a part of the first surface 5701 of the n-th light-emitting structure 570, and then the first light-emitting structure 520, the second light-emitting structure 540, ..., and the n-th light-emitting structure 570 may be electrically connected to the substrate 601 by means of the electrical connection structure such as the soldering members 615, 620, 625, 630, 635, and 640.

Referring to FIG. 9, the configuration has been described in which at least one of the insulators (e.g., the first insulator 920, the second insulator 930, ..., and the n-th insulator 940) is disposed on the first surface (e.g., the first surface 5201 of the first light-emitting structure 520, the first surface 5401 of the second light-emitting structure 540, ..., and the first surface 5701 of the n-th light-emitting structure 570) of each of the light-emitting structures. However, the disclosure is not limited thereto. For example, instead of the configuration in which at least one of the insulators (e.g., the first insulator 920, the second insulator 930, ..., and the n-th insulator 940) is disposed on the first surface of each of the light-emitting structures, at least one of the insulators may be disposed on the second surface (e.g., the second surface 5202 of the first light-emitting structure 520, the second surface 5402 of the second light-emitting structure 540, ..., and the second surface 5702 of the n-th light-emitting structure 570) of each of the light-emitting structures.

As described above with reference to FIG. 9 according to various embodiments, the insulators 920, 930, and 940 are disposed between the plurality of light sources included in each of the light-emitting structures, such that the plurality of light sources may be insulated, a short circuit may be prevented, the light-emitting structures may be securely fixed to the substrate 601, and structural rigidity may be improved.

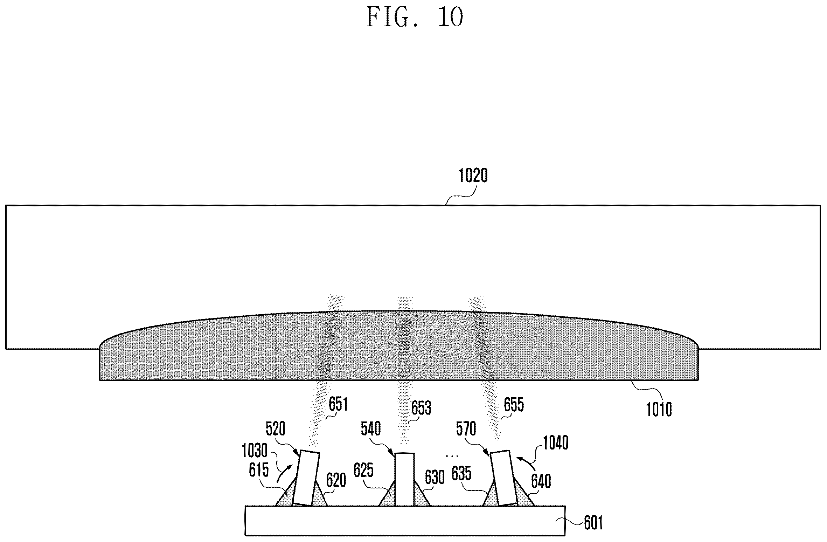

FIG. 10 is a view for explaining a method of measuring a plurality of pieces of bio-information of a user by using a plurality of light sources included in at least one of light-emitting structures according to an embodiment of the disclosure.

Referring to FIGS. 6A, 6B, and 7 to 9, the configuration has been described in which in a state in which at least one of the light-emitting structures (e.g., first light-emitting structure 520, second light-emitting structure 540, ..., and n-th light-emitting structure 570 in FIG. 6A) of the first biometric sensor (e.g., the first biometric sensor 430 in FIG. 4) is not inclined (e.g., a state perpendicular to the substrate (e.g., substrate 601 in FIG. 6A)), at least one of the light-emitting structures is electrically connected to the substrate 601 by means of the soldering (e.g., the soldering members (e.g., soldering by means of the first soldering member 615, the second soldering member 620, the third soldering member 625, the fourth soldering member 630, the fifth soldering member 635, and/or the sixth soldering member 640 in FIGS. 6A and 6B) or the wires (e.g., the first wire 720, the second wire 730, and/or the third wire 740 in FIG. 7). However, the disclosure is not limited thereto.

For example, referring to FIG. 10, in a state in which a first light-emitting structure 520, a second light-emitting structure 540, ..., and an n-th light-emitting structure 570 of a first biometric sensor 430 are inclined at a particular angle, the first light-emitting structure 520, the second light-emitting structure 540, ..., and the n-th light-emitting structure 570 may be electrically connected to a substrate 601 by means of the soldering (or wires).

In the embodiment, the electronic device 200 may include a rear surface plate 1010 (e.g., the rear plate 493 in FIG. 4). In case that the electronic device 200 is worn on (or fixed to) of a part of the user's body (e.g., the wrist), at least a part of the rear surface plate 1010 may be brought into contact with a part of the user's body (e.g., the wrist).

In the embodiment, at least one of the first light-emitting structure 520, the second light-emitting structure 540, ..., and the n-th light-emitting structure 570 may be electrically connected to the substrate 601 by means of the soldering (or wires) in a state in which at least one of the light-emitting structures is inclined at the particular angle so that the light emitted from the plurality of light sources included in each of the light-emitting structures (e.g., the first light-emitting structure 520, the second light-emitting structure 540, ..., and the n-th light-emitting structure 570) reaches a light irradiation area 1020 (e.g., skin of a part of the user's body (e.g., the wrist)) (e.g., the light emitted from each of the light sources is focused on the light irradiation area 1020).

In the embodiment, the particular angle at which at least one of the first light-emitting structure 520, the second light-emitting structure 540, ..., and the n-th light-emitting structure 570 is inclined may be set on the basis of a distance between each of the light-emitting structures and the light irradiation area 1020.

However, the disclosure is not limited thereto. The particular angle at which at least one of the first light-emitting structure 520, the second light-emitting structure 540, ..., and the n-th light-emitting structure 570 is inclined may be set in consideration of the distance between each of the light-emitting structures and the light irradiation area 1020 and a configuration in which the light is reflected by the rear surface plate 1010 between each of the light-emitting structures and the light irradiation area 1020.

For example, the first light-emitting structure 520 may be inclined at a particular angle (e.g., about 0.1 to 10 degrees) in consideration of a distance between the first light-emitting structure 520 and the light irradiation area 1020 and a configuration in which the light is reflected by the rear surface plate 1010 between the first light-emitting structure 520 and the light irradiation area 1020, as inclined at 1030. In addition, the second light-emitting structure 540 may be kept in a non-inclined state (e.g., the state perpendicular to the substrate 601) in consideration of a distance between the second light-emitting structure 540 and the light irradiation area 1020 and a configuration in which the light is reflected by the rear surface plate 1010 between the second light-emitting structure 540 and the light irradiation area 1020. In addition, the n-th light-emitting structure 570 may be inclined at a particular angle (e.g., about 0.1 to 10 degrees) in consideration of a distance between the n-th light-emitting structure 570 and the light irradiation area 1020 and a configuration in which the light is reflected by the rear surface plate 1010 between the n-th light-emitting structure 570 and the light irradiation area 1020, as inclined at 1040. In other words, the soldering (or wiring) may be performed so that the first light-emitting structure 520, the second light-emitting structure 540, ..., and the n-th light-emitting structure 570 may be electrically connected to the substrate 601 in the state in which the first light-emitting structure 520 and the n-th light-emitting structure 570 are inclined at the particular angle (e.g., about 0.1 to 10 degrees) and the second light-emitting structure 540 is not inclined at the particular angle (e.g., the state perpendicular to the substrate 601).

In the embodiment, when an input (or signal) for measuring a bio signal is detected, the light sources included in the first light-emitting structure 520, the light sources included in the second light-emitting structure 540, and the light sources included in the n-th light-emitting structure 570 may sequentially emit light in different wavelength ranges through different optical paths. For example, the processor 120 may allow the plurality of light sources included in the first light-emitting structure 520 inclined at 1030 at a particular angle (e.g., about 0.1 to 10 degrees) to sequentially emit light at the particular angle. The plurality of light sources included in the first light-emitting structure 520 may sequentially emit light in different wavelength ranges through different optical paths. The light sequentially emitted in the different wavelength ranges through the different optical paths may reach the light irradiation area 1020. The processor 120 may allow the plurality of light sources included in the second light-emitting structure 540 disposed at an angle in the state perpendicular to the substrate 601 to sequentially emit light at the angle in the state perpendicular to the substrate 601. The plurality of light sources included in the second light-emitting structure 540 may sequentially emit light in different wavelength ranges through different optical paths (e.g., optical paths different from those of the plurality of light sources included in the first light-emitting structure 520). The light sequentially emitted in the different wavelength ranges through the different optical paths may reach the light irradiation area 1020. The processor 120 may allow the plurality of light sources included in the n-th light-emitting structure 570 inclined at 1040 at a particular angle (e.g., about 0.1 to 10 degrees) to sequentially emit light at the particular angle. The plurality of light sources included in the n-th light-emitting structure 570 may sequentially emit light in different wavelength ranges through different optical paths (e.g., optical paths different from those of the plurality of light sources included in the first and second light-emitting structures 520 and 540). The light sequentially emitted in the different wavelength ranges through the different optical paths may reach the light irradiation area 1020.

In the embodiment, the light sources included in the first light-emitting structure 520, the light sources included in the second light-emitting structure 540, and the light sources included in the n-th light-emitting structure 570 may sequentially emit light in different wavelength ranges through different optical paths, thereby acquiring various pieces of bio-information.

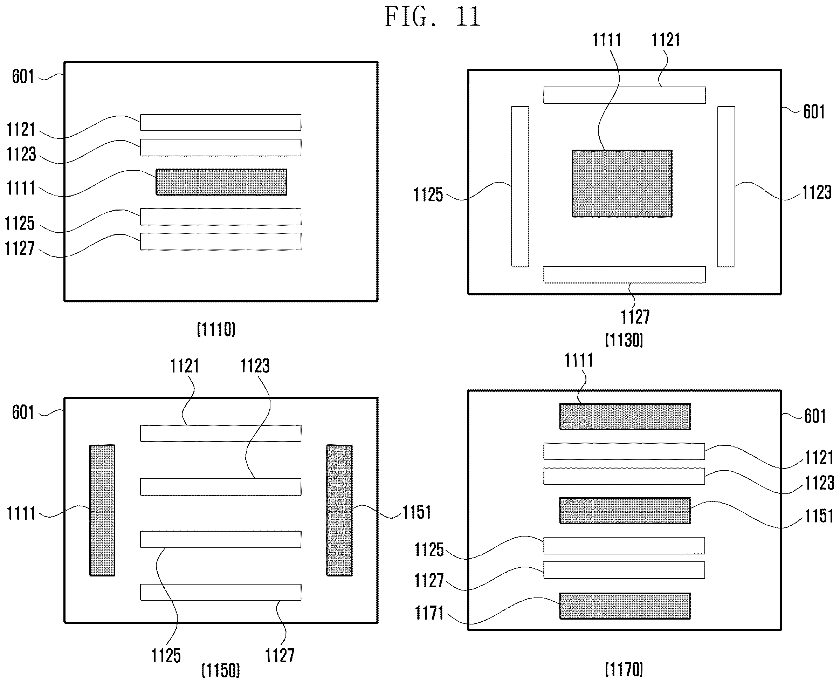

FIG. 11 is a view for explaining a monitoring circuit according to an embodiment of the disclosure.

Referring to FIG. 11, an electronic device (e.g., electronic device 101 in FIG. 1 or electronic device 200 in FIGS. 2 to 4) may include at least one monitoring circuit configured to identify the intensity (or amount) and wavelength range of the light emitted from the plurality of light sources included in each of the light-emitting structures of the first biometric sensor (e.g., the first biometric sensor 430 in FIG. 4). At least one monitoring circuit may be disposed on the substrate (e.g., substrate 601 in FIG. 6). For example, at least one monitoring circuit may be disposed in the vicinity of at least one light-emitting structure disposed on the substrate 601.

The description will be made on the assumption that four light-emitting structures are disposed on the substrate 601 in FIG. 11 according to various embodiments. For example, the four light-emitting structures may include a first light-emitting structure 1121 (e.g., the first light-emitting structure 520 in FIG. 5), a second light-emitting structure 1123 (e.g., the second light-emitting structure 540 in FIG. 6A), a third light-emitting structure 1125, and a fourth light-emitting structure 1127.

Referring to reference numeral <1110> according to the embodiment, the first light-emitting structure 1121, the second light-emitting structure 1123, the third light-emitting structure 1125, the fourth light-emitting structure 1127, and a first monitoring circuit 1111 may be disposed on the substrate 601. For example, the first light-emitting structure 1121, the second light-emitting structure 1123, the third light-emitting structure 1125, and the fourth light-emitting structure 1127 may be disposed in parallel on the substrate 601. The first monitoring circuit 1111 may be disposed between the second light-emitting structure 1123 and the third light-emitting structure 1125 and identify the intensity (or amount) and wavelength range of the light sequentially emitted from the plurality of light sources included in each of the light-emitting structures 1121, 1123, 1125, and 1127.

However, the disclosure is not limited thereto. Referring to reference numeral <1130>, the first light-emitting structure 1121, the second light-emitting structure 1123, the third light-emitting structure 1125, and the fourth light-emitting structure 1127 may be disposed on an outer periphery of the substrate 601 while surrounding the substrate 601. The first monitoring circuit 1111 may be disposed in a central area of the substrate 601 and identify the intensity (or amount) and wavelength range of the light sequentially emitted from the plurality of light sources included in each of the light-emitting structures 1121, 1123, 1125, and 1127.

Referring to reference numeral <1150> according to the embodiment, the first light-emitting structure 1121, the second light-emitting structure 1123, the third light-emitting structure 1125, the fourth light-emitting structure 1127, the first monitoring circuit 1111, and a second monitoring circuit 1151 may be disposed on the substrate 601. For example, the first light-emitting structure 1121, the second light-emitting structure 1123, the third light-emitting structure 1125, and the fourth light-emitting structure 1127 may be disposed in parallel on the substrate 601. The first monitoring circuit 1111 and the second monitoring circuit 1151 may be disposed on the outer periphery of the substrate 601 and disposed in a direction orthogonal to the light-emitting structures. The first monitoring circuit 1111 may identify the intensity (or amount) and wavelength range of the light sequentially emitted from the plurality of light sources included in at least two of the first light-emitting structure 1121, the second light-emitting structure 1123, the third light-emitting structure 1125, and the fourth light-emitting structure 1127. The second monitoring circuit 1151 may identify the intensity (or amount) and wavelength range of the light sequentially emitted from the plurality of light sources included in at least two of the remaining light-emitting structures among the first light-emitting structure 1121, the second light-emitting structure 1123, the third light-emitting structure 1125, and the fourth light-emitting structure 1127.

Referring to reference numeral <1170> according to the embodiment, the first light-emitting structure 1121, the second light-emitting structure 1123, the third light-emitting structure 1125, the fourth light-emitting structure 1127, the first monitoring circuit 1111, the second monitoring circuit 1151, and a third monitoring circuit 1171 may be disposed on the substrate 601. For example, the first light-emitting structure 1121, the second light-emitting structure 1123, the third light-emitting structure 1125, and the fourth light-emitting structure 1127 may be disposed in parallel on the substrate 601. When the substrate 601 is viewed from the front side, the first monitoring circuit 1111 may be disposed above the first light-emitting structure 1121, the second monitoring circuit 1151 may be disposed between the second light-emitting structure 1123 and the third light-emitting structure 1125, and the third monitoring circuit 1171 may be disposed below the fourth light-emitting structure 1127. In this case, the first monitoring circuit 1111 may identify the intensity (or amount) and wavelength range of the light sequentially emitted from the plurality of light sources included in the first light-emitting structure 1121. The second monitoring circuit 1151 may identify the intensity (or amount) and wavelength range of the light sequentially emitted from the plurality of light sources included in the second light-emitting structure 1123 and the third light-emitting structure 1125. The third monitoring circuit 1171 may identify the intensity (or amount) and wavelength range of the light sequentially emitted from the plurality of light sources included in the fourth light-emitting structure 1127.

In the embodiment, at least one of the monitoring circuits (e.g., the first monitoring circuit 1111, the second monitoring circuit 1151, and/or the third monitoring circuit 1171) may identify whether the plurality of light sources included in the light-emitting structures 1121, 1123, 1125, and 1127 emits light with designated intensities (or power) and in designated wavelength ranges and transfer the identified information (e.g., data related to whether the light is outputted with the designated intensities (or power) and in the designated wavelengths) to the processor (e.g., the processor 120 in FIG. 1). The processor 120 may control the intensity (or amount) and wavelength range of the emitted light by adjusting gains on the basis of the information transferred through at least one of the monitoring circuits (e.g., the first monitoring circuit 1111, the second monitoring circuit 1151, and/or the third monitoring circuit 1171).

In the embodiment, the electronic device 200 may include a light detection circuit (not illustrated). The light detection circuit may receive the light emitted from the plurality of light sources included in the light-emitting structures 1121, 1123, 1125, and 1127. For example, the light detection circuit may sequentially receive the light sequentially emitted from the plurality of light sources. For example, the plurality of light sources having different wavelength ranges may sequentially emit light to the light irradiation area (e.g., the light irradiation area 1020 in FIG. 10) under the control of the processor 120. The light detection circuit may sequentially receive the light that is sequentially emitted from the plurality of light sources and reflected by the light irradiation area 1020. The processor 120 may measure (or acquire or identify) a plurality of pieces of bio-information on the basis of the amount (or intensity) and wavelength range of the light received by the light detection circuit.

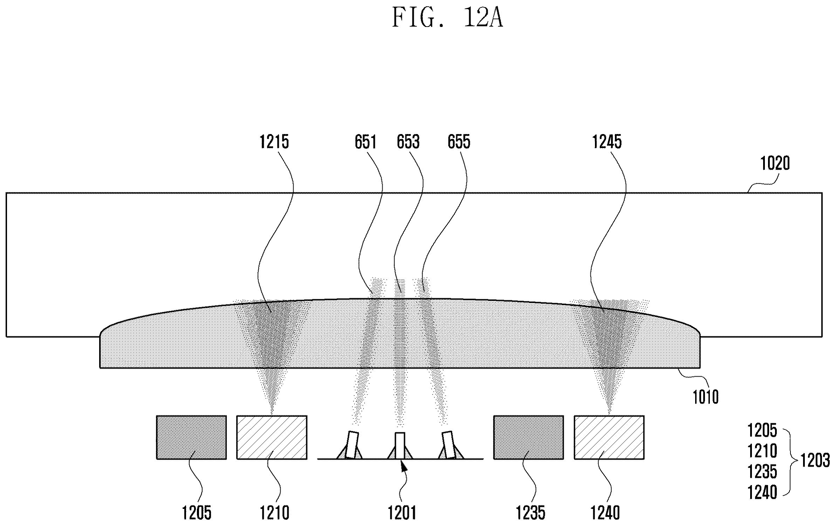

FIG. 12A is a view illustrating a state in which a plurality of biometric sensors is disposed according to an embodiment of the disclosure.

Referring to FIG. 12A, an electronic device (e.g., electronic device 101 in FIG. 1 or electronic device 200 in FIGS. 2 to 4) may include a first biometric sensor 1201 (e.g., the first biometric sensor 430 in FIG. 4) and a second biometric sensor 1203.

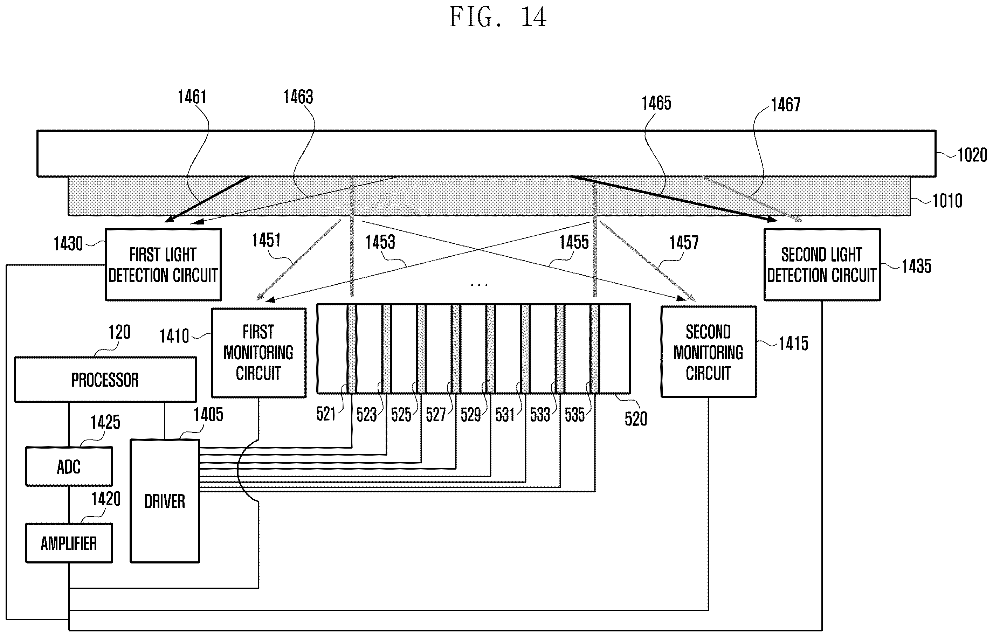

In the embodiment, the first biometric sensor 1201 may include sensors configured to acquire (or receive) light by means of at least one of the light-emitting structures (e.g., the first light-emitting structure 520, the second light-emitting structure 540, ..., and the n-th light-emitting structure 570 in FIG. 5), at least one of the monitoring circuits (e.g., the first monitoring circuit 1111, the second monitoring circuit 1151, and/or the third monitoring circuit 1171 in FIG. 11), and/or the light detection circuits (e.g., a first light detection circuit 1430 and a second light detection circuit 1435 in FIG. 14) that have been described above with reference to FIGS. 5, 6A, 6B, and 7 to 11. In the embodiment, the plurality of light sources included in at least one of the light-emitting structures may include laser diodes.

In the embodiment, the second biometric sensor 1203 may include a photoplethysmography (PPG) sensor. For example, the second biometric sensor 1203 may include light-emitting sensors 1210 and 1240 configured to emit light, and light-receiving sensors 1205 and 1235 configured to receive light. In the embodiment, the light-emitting sensors 1210 and 1240 may include light-emitting diodes (LEDs), and the light-receiving sensors 1205 and 1235 may include photo-diodes (PDs). However, the disclosure is not limited thereto.

In the embodiment, the first biometric sensor 1201 and the second biometric sensor 1203 may be disposed on the substrate (e.g., the substrate 601 in FIG. 6A). For example, the first biometric sensor 1201 and the second biometric sensor 1203 may be disposed on the same plane of the substrate 601.

In the embodiment, the electronic device 200 may include the rear surface plate 1010 (e.g., the rear plate 493 in FIG. 4). In case that the electronic device 200 is worn on (or fixed to) of a part of the user's body (e.g., the wrist), at least a part of the rear surface plate 1010 may be brought into contact with a part of the user's body (e.g., the wrist).

In the embodiment, when an input (or signal) for measuring a bio signal is detected, the processor (e.g., processor 120 in FIG. 1) may perform control so that the light 651, 653, and 655 is emitted from the plurality of light sources included in at least one of the light-emitting structures (e.g., the first light-emitting structure 520, the second light-emitting structure 540, ..., and the n-th light-emitting structure 570 in FIG. 5) included in the first biometric sensor 1201. The light 651, 653, and 655 sequentially emitted from the plurality of light sources included in at least one of the light-emitting structures (e.g., the first light-emitting structure 520, the second light-emitting structure 540, ..., and the n-th light-emitting structure 570 in FIG. 5) of the first biometric sensor 1201 may be acquired (or received) by the monitoring circuits (e.g., a first monitoring circuit 1410 and a second monitoring circuit 1415) and/or the light detection circuits (e.g., the first light detection circuit 1430 and the second light detection circuit 1435). The processor 120 may generate a spectrum for each wavelength range on the basis of the light acquired (or received) by the monitoring circuits (e.g., the first monitoring circuit 1410 and the second monitoring circuit 1415) and/or the light detection circuits (e.g., the first light detection circuit 1430 and the second light detection circuit 1435). The processor 120 may measure (or acquire) at least one first bio-information on the basis of the generated spectrum for each wavelength range.

In the embodiment, when an input (or signal) for measuring a bio signal is detected, the processor 120 may perform control so that light 1215 and 1245 is emitted from the light-emitting sensors 1210 and 1240 included in the second biometric sensor 1203. The light 1215 and 1245 emitted from the light-emitting sensors 1210 and 1240 of the second biometric sensor 1203 may be reflected by the light irradiation area 1020 (e.g., skin of a part of the user's body (e.g., the wrist)). The light-receiving sensors 1205 and 1235 may receive the light reflected from the light irradiation area 1020. The processor 120 may measure (or acquire) at least one second bio-information of the user on the basis of the light received by the light-receiving sensors 1205 and 1235.

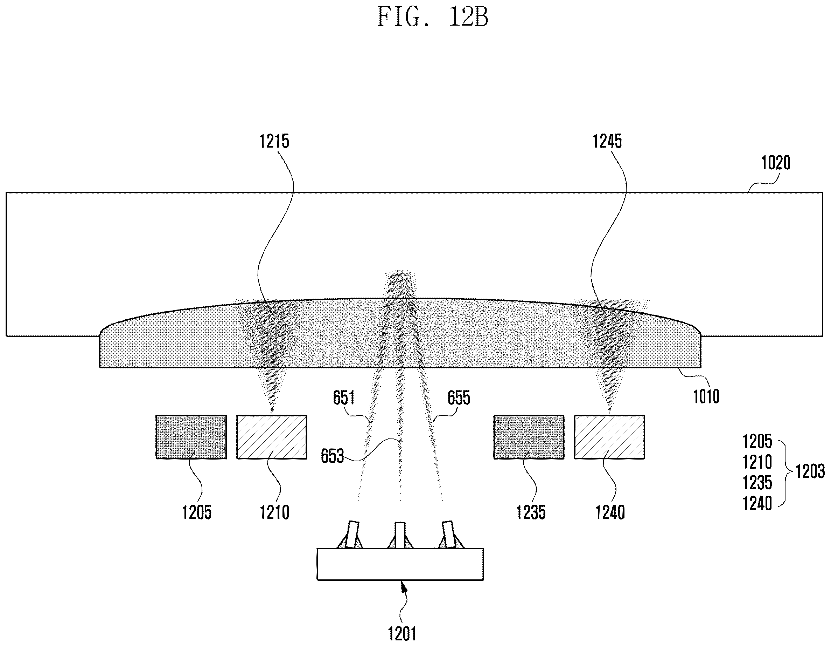

Referring to FIG. 12A, the configuration has been described in which a first biometric sensor 1201 and a second biometric sensor 1203 are disposed on the same plane of a substrate 601. However, the disclosure is not limited thereto. In this regard, various embodiments will be described below with reference to FIG. 12B.

FIG. 12B is a view illustrating a state in which a plurality of biometric sensors is disposed according to an embodiment of the disclosure.

Referring to FIG. 12B, an electronic device (e.g., electronic device 101 in FIG. 1 or electronic device 200 in FIGS. 2 to 4) may include a first biometric sensor 1201 (e.g., the first biometric sensor 430 in FIG. 4) and a second biometric sensor 1203.

Because the first biometric sensor 1201 and the second biometric sensor 1203 in FIG. 12B according to various embodiments are substantially identical to the first biometric sensor 1201 and the second biometric sensor 1203 in FIG. 12A described above, a detailed description thereof will be substituted with the description described with reference to FIG. 12A.

In the embodiment, the first biometric sensor 1201 and the second biometric sensor 1203 may be disposed on the substrate (e.g., the substrate 601 in FIG. 6A). For example, the first biometric sensor 1201 and the second biometric sensor 1203 may be disposed on different planes of the substrate 601. For example, a groove may be formed in a partial area of the substrate 601, the first biometric sensor 1201 may be disposed in the formed groove, and the second biometric sensor 1203 may be disposed in another partial area of the substrate 601.

As another example, the first biometric sensor 1201 and the second biometric sensor 1203 may be disposed on different substrates. For example, the first biometric sensor 1201 may be disposed on the substrate 601, and the second biometric sensor 1203 may be disposed on the printed circuit board 480 illustrated in FIG. 4. In this case, a partial area of the printed circuit board 480, which overlaps the first biometric sensor 1201, may be made of a material capable of transmitting light (e.g., infrared rays or visible rays). However, the disclosure is not limited thereto. The partial area of the printed circuit board 480, which overlaps the first biometric sensor 1201, may have a hole formed so that the light emitted from the first biometric sensor 1201 may reach the light irradiation area 1020.

Referring to FIG. 12B, a distance between a rear surface plate 1010 (e.g., the rear plate 493 in FIG. 4) and a second biometric sensor 1203 may be longer than a distance between the rear surface plate 1010 and a first biometric sensor 1201. For example, the plurality of light sources included in the second biometric sensor 1203 may include laser diodes. The laser diode has straightness and is less likely to disperse light. Therefore, the second biometric sensor 1203 may be disposed to be farther from the rear surface plate 1010 than the first biometric sensor 1201 from the rear surface plate 1010.

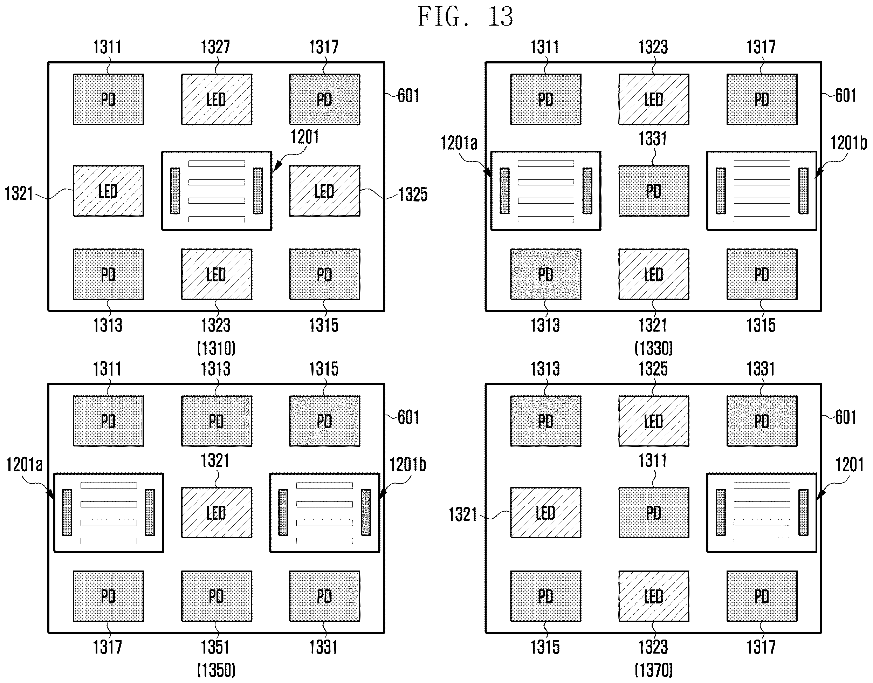

FIG. 13 is a view illustrating a state in which a plurality of biometric sensors is disposed according to an embodiment of the disclosure.

Referring to FIG. 13, an electronic device (e.g., electronic device 101 in FIG. 1 or electronic device 200 in FIGS. 2 to 4) may include a plurality of biometric sensors. The plurality of biometric sensors may include a first biometric sensor (e.g., the first biometric sensor 430 in FIG. 4 and the first biometric sensor 1201 in FIGS. 12A and 12B) and a second biometric sensor (e.g., the second biometric sensor 1203 in FIGS. 12A and 12B).

Because the first biometric sensor 1201 (or a first-first biometric sensor 1201a and a first-second biometric sensor 1201b) and the second biometric sensor in FIG. 13 according to various embodiments are substantially identical to the first biometric sensor 1201 and the second biometric sensor 1203 in FIG. 12A described above, a detailed description thereof will be substituted with the description described with reference to FIG. 12A.

Referring to FIG. 13, in case that an electronic device 200 includes the plurality of biometric sensors, the plurality of biometric sensors may be disposed in the same way illustrated in reference numeral <1310>, <1330>, <1350>, or <1370>.

The description will be made on the assumption that the plurality of biometric sensors is disposed on the same substrate (e.g., the substrate 601 in FIG. 6A) in FIG. 13 according to various embodiments.

Referring to reference numeral <1310> according to the embodiment, the electronic device 200 may include the first biometric sensor 1201 and the second biometric sensors, e.g., the light-emitting sensors (e.g., a first light-emitting sensor 1321, a second light-emitting sensor 1323, a third light-emitting sensor 1325, and a fourth light-emitting sensor 1327) (e.g., LEDs) configured to emit light, and the light-receiving sensors (e.g., a first light-receiving sensor 1311, a second light-receiving sensor 1313, a third light-receiving sensor 1315, and a fourth light-receiving sensor 1317) (e.g., PDs) configured to receive light. For example, the first biometric sensor 1201 may be disposed in the central area of the substrate 601, and the light-emitting sensors (e.g., the first light-emitting sensor 1321, the second light-emitting sensor 1323, the third light-emitting sensor 1325, and the fourth light-emitting sensor 1327) and the light-receiving sensors (e.g., the first light-receiving sensor 1311, the second light-receiving sensor 1313, the third light-receiving sensor 1315, and the fourth light-receiving sensor 1317) may be disposed to surround the first biometric sensor 1201.

Referring to reference numeral <1330> according to the embodiment, the electronic device 200 may include the first-first biometric sensor 1201a, the first-second biometric sensor 1201b, and the second biometric sensors, e.g., the light-emitting sensors (e.g., the first light-emitting sensor 1321 and the second light-emitting sensor 1323) (e.g., LEDs) configured to emit light and the light-receiving sensors (e.g., the first light-receiving sensor 1311, the second light-receiving sensor 1313, the third light-receiving sensor 1315, the fourth light-receiving sensor 1317, a fifth light-receiving sensor 1331) (e.g., PDs) configured to receive light. For example, the fifth light-receiving sensor 1331 may be disposed at the center of the substrate 601, and the first-first biometric sensor 1201a, the first-second biometric sensor 1201b, the light-emitting sensors (e.g., the first light-emitting sensor 1321 and the second light-emitting sensor 1323), and the light-receiving sensors (e.g., the first light-receiving sensor 1311, the second light-receiving sensor 1313, the third light-receiving sensor 1315, and the fourth light-receiving sensor 1317) may be disposed to surround the fifth light-receiving sensor 1331.

Referring to reference numeral <1350> according to the embodiment, the electronic device 200 may include the first-first biometric sensor 1201a, the first-second biometric sensor 1201b, and the second biometric sensors, e.g., the light-emitting sensor (e.g., the first light-emitting sensor 1321) (e.g., an LED) configured to emit light and the light-receiving sensors (e.g., the first light-receiving sensor 1311, the second light-receiving sensor 1313, the third light-receiving sensor 1315, the fourth light-receiving sensor 1317, the fifth light-receiving sensor 1331, and a sixth light-receiving sensor 1351) (e.g., PDs) configured to receive light. For example, the first light-emitting sensor 1321 may be disposed at the center of the substrate 601, and the first-first biometric sensor 1201a, the first-second biometric sensor 1201b, and the light-receiving sensors (e.g., the first light-receiving sensor 1311, the second light-receiving sensor 1313, the third light-receiving sensor 1315, the fourth light-receiving sensor 1317, the fifth light-receiving sensor 1331, and the sixth light-receiving sensor 1351) may be disposed to surround the first light-emitting sensor 1321.

Referring to reference numeral <1370> according to the embodiment, the electronic device 200 may include the first biometric sensor 1201 and the second biometric sensors, e.g., the light-emitting sensors (e.g., the first light-emitting sensor 1321, the second light-emitting sensor 1323, and the third light-emitting sensor 1325) (e.g., LEDs) configured to emit light and the light-receiving sensors (e.g., the first light-receiving sensor 1311, the second light-receiving sensor 1313, the third light-receiving sensor 1315, the fourth light-receiving sensor 1317, and the fifth light-receiving sensor 1331) (e.g., PDs) configured to receive light. For example, the first light-receiving sensor 1311 may be disposed at the center of the substrate 601, and the first biometric sensor 1201, the light-emitting sensors (e.g., the first light-emitting sensor 1321, the second light-emitting sensor 1323, and the third light-emitting sensor 1325), and the light-receiving sensors (e.g., the second light-receiving sensor 1313, the third light-receiving sensor 1315, the fourth light-receiving sensor 1317, and the fifth light-receiving sensor 1331) may be disposed to surround the first light-receiving sensor 1311.

FIG. 14 is a view for explaining a method of measuring a plurality of pieces of bio-information by using an electronic device including at least one light-emitting structure according to an embodiment of the disclosure.

Referring to FIG. 14, an electronic device (e.g., electronic device 101 in FIG. 1 or electronic device 200 in FIGS. 2 to 4) may include a processor (e.g., processor 120 in FIG. 1), a driver (e.g., laser diode (LD) driver) 1405, a first monitoring circuit 1410, a second monitoring circuit 1415, an amplifier (e.g., a trans-impedance amplifier (TIA)) 1420, an analog-digital converter (ADC) 1425, a first light detection circuit 1430, a second light detection circuit 1435, and at least one of the light-emitting structures (e.g., the first light-emitting structure 520, the second light-emitting structure 540, ..., and the n-th light-emitting structure 570 in FIG. 6A).

In the embodiment, the processor 120 may operate the driver 1405 and process digital signals received from the ADC 1425. For example, the processor 120 may control the driver 1405 to adjust the current to be supplied to the plurality of light sources on the basis of an outside temperature and/or signals of the monitoring circuits (e.g., the first monitoring circuit 1410 and the second monitoring circuit 1415).

In the embodiment, the driver 1405 may control operations of the plurality of light sources having different wavelength ranges and included in the first light-emitting structure 520 under the control of the processor 120. For example, the driver 1405 may turn on or off the plurality of light sources included in the first light-emitting structure 520 under the control of the processor 120. For example, the driver 1405 may perform control to supply the current to the first light-emitting structure 520 and allow the plurality of light sources to sequentially emit light in the corresponding wavelength range under the control of the processor 120. However, the disclosure is not limited thereto. The driver 1405 may modulate the current to acquire an additional signal-to-noise ratio (SNR) under the control of the processor 120.

In the embodiment, the amplifier 1420 may convert the current, which is generated by the monitoring circuits (e.g., the first monitoring circuit 1410 and the second monitoring circuit 1415), into voltages and transmit the voltages to the ADC 1425. In this case, the amplifier 1420 may adjust gains for converting the current into the voltage in consideration of the range and/or signal intensity of the ADC 1425. However, the disclosure is not limited thereto. The amplifier 1420 may adjust the bias so that the monitoring circuits (e.g., the first monitoring circuit 1410 and the second monitoring circuit 1415) may operate within designated ranges.

In the embodiment, the ADC 1425 may change the voltage, which is received from the amplifier 1420, into digital signals so that the processor 120 may recognize the digital signals.

In the embodiment, the processor 120 may sequentially emit light from the plurality of light sources included in each of the light-emitting structures having different wavelength ranges and measure at least a part of the light reflected by the rear surface plate 1010 and/or the light irradiation area 1020 (e.g., skin of a part of the user's body (e.g., the wrist)). The processor 120 may generate a spectrum for each wavelength range on the basis of the amount of absorbed light calculated based on the reflected light for each wavelength range.

For example, in case that the light in the first-first wavelength range (e.g., λ_{1,1}) is emitted from the first-first light source 521 of the first light-emitting structure 520, the monitoring circuits (e.g., the first monitoring circuit 1410 and the second monitoring circuit 1415) may acquire (or receive) at least a part of light 1451, 1453, 1455, and 1457 reflected or scattered by the rear surface plate 1010. In addition, in case that the light in the first-first wavelength range (e.g., λ_{1,1}) is emitted from the first-first light source of the first light-emitting structure 520, the light detection circuits (e.g., the first light detection circuit 1430 and the second light detection circuit 1435) may acquire (or receive) light 1461, 1463, 1465, and 1467 absorbed and reflected by the light irradiation area 1020 (e.g., skin of a part of the user's body (e.g., the wrist)).

Although not illustrated in FIG. 14 according to various embodiments, the electronic device 200 may further include the second light-emitting structure 540, ..., and the n-th light-emitting structure 570.

In the embodiment, the processor 120 may sequentially emit light in the first-second wavelength range (e.g., λ_{1,2}) from the first-second light source 523, emit light in the first-third wavelength range (e.g., λ_{1,3}) from the first-third light source 525, emit light in the first-fourth wavelength range (e.g., λ_{1,4}) from the first-fourth light source 527, emit light in the first-fifth wavelength range (e.g., λ_{1,5}) from the first-fifth light source 529, emit light in the first-sixth wavelength range (e.g., λ_{1,6}) from the first-sixth light source 531, emit light in the first-seventh wavelength range (e.g., λ_{1,7}) from the first-seventh light source 533, and emit light in the first-eighth wavelength range (e.g., λ_{1,8}) from the first-eighth light source 535.

In the embodiment, the processor 120 may allow the monitoring circuits (e.g., the first monitoring circuit 1410 and the second monitoring circuit 1415) to acquire (or receive) the light emitted from the first-second light source 523 to the first-eighth light source 535 and reflected or scattered by the rear surface plate 1010. The processor 120 may allow the light detection circuits (e.g., the first light detection circuit 1430 and the second light detection circuit 1435) to acquire (or receive) the light emitted from the first-second light source 523 to the first-eighth light source 535 and absorbed and reflected by the light irradiation area 1020. The processor 120 may generate a spectrum for each of the wavelength ranges (e.g., the first-first wavelength range (e.g., λ_{1,1}) to the first-eighth wavelength range (e.g., λ_{1,8})) based on the light acquired (or received) by the monitoring circuits (e.g., the first monitoring circuit 1410 and the second monitoring circuit 1415) and the light acquired (or received) by the light detection circuits (e.g., the first light detection circuit 1430 and the second light detection circuit 1435).

In the embodiment, the processor 120 may allow the plurality of light sources included in the other light-emitting structures, e.g., the second light-emitting structure 540 to emit the light 653 in the second-first wavelength range (e.g., λ_{2,1}) to the second-eighth wavelength range (e.g., λ_{2,8}). In addition, the processor 120 may allow the plurality of light sources included in the other light-emitting structures, e.g., the n-th light-emitting structure 570 to emit the light 655 in the n-th-first wavelength range (e.g., λ_{n,1}) to the n-th-eighth wavelength range (e.g., λ_{n,8}).

In the embodiment, the processor 120 may allow the monitoring circuits (e.g., the first monitoring circuit 1410 and the second monitoring circuit 1415) to acquire (or receive) at least a part of the light emitted from the plurality of light sources included in the second light-emitting structure 540 and/or the plurality of light sources included in the n-th light-emitting structure 570 and reflected or scattered by the rear surface plate 1010. The processor 120 may allow the light detection circuits (e.g., the first light detection circuit 1430 and the second light detection circuit 1435) to acquire (or receive) the light emitted from the plurality of light sources included in the second light-emitting structure 540 and/or the plurality of light sources included in the n-th light-emitting structure 570 and absorbed and reflected by the light irradiation area 1020. The processor 120 may generate a spectrum for each of the wavelength ranges (e.g., the second-first wavelength range (e.g., λ_{2,1}) to the second-eighth wavelength range (e.g., λ_{2,8}) and the n-th-first wavelength range (e.g., λ_{n,1}) to the n-th-eighth wavelength range (e.g., λ_{n,8})) on the basis of the light acquired (or received) by the monitoring circuits (e.g., the first monitoring circuit 1410 and the second monitoring circuit 1415) and the light acquired (or received) by the light detection circuits (e.g., the first light detection circuit 1430 and the second light detection circuit 1435).

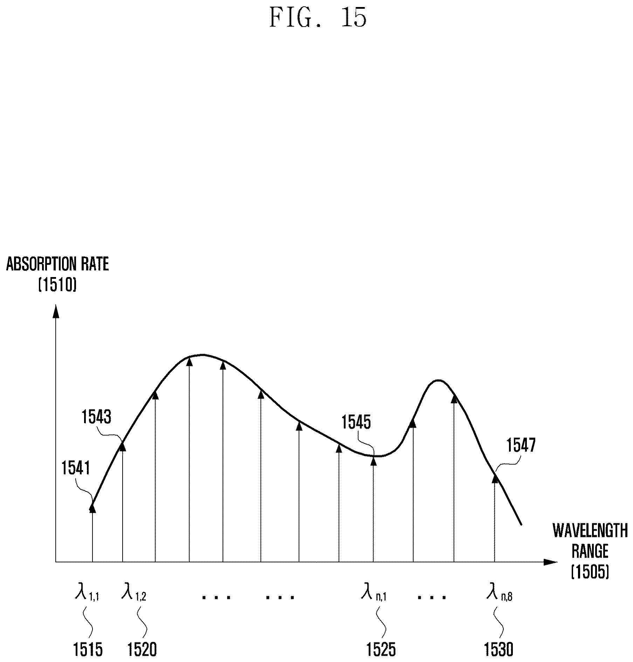

In the embodiment, the processor 120 may measure at least one bio-information by analyzing the spectrum for each wavelength range. The configuration for generating the spectrum for each wavelength range according to the embodiment will be described in detail with reference to FIG. 15 to be described below.

FIG. 15 is a view for explaining a spectrum graph related to bio-information according to an embodiment of the disclosure.

Referring to FIG. 15, the x-axis means a wavelength range (nanometers (nm)) 1505, and the y-axis means an absorption rate 1510.

Referring to FIG. 15, a processor (e.g., processor 120 in FIG. 1) of an electronic device (e.g., electronic device 101 in FIG. 1 or electronic device 200 in FIGS. 2 to 4) may sequentially emit light from a plurality of light sources included in light-emitting structures (e.g., the first light-emitting structure 520, the second light-emitting structure 540, ..., and the n-th light-emitting structure 570 in FIG. 6A) having different wavelength ranges and measure the light reflected (and/or the light scattered) by a rear surface plates (e.g., the rear surface plate 1010 in FIG. 10) and/or the light irradiation area (e.g., the light irradiation area 1020 in FIG. 10) (e.g., skin of a part of the user's body (e.g., the wrist)).

For example, in case that the light in a first-first wavelength range 1515 (e.g., λ_{1,1}) is emitted from the first-first light source 521 of the light-emitting structure 520, the monitoring circuits (e.g., the first monitoring circuit 1410 and the second monitoring circuit 1415 in FIG. 14) may acquire (or receive) at least a part of the light (e.g., 1451, 1453, 1455, and 1457 in FIG. 14) reflected or scattered by the rear surface plate 1010. In addition, in case that the light in the first-first wavelength range 1515 (e.g., λ_{1,1}) is emitted from the first-first light source 521 of the light-emitting structure 520, the light detection circuits (e.g., the first light detection circuit 1430 and the second light detection circuit 1435 in FIG. 14) may acquire (or receive) the light (e.g., 1461, 1463, 1465, and 1467 in FIG. 14) absorbed and reflected by the light irradiation area 1020 (e.g., skin of a part of the user's body (e.g., the wrist)). The description will be made on the assumption that the amount (or intensity) of light 1451, 1453, 1455, and 1457 reflected or scattered by the rear surface plate 1010 is defined as M_{1,1}, and the amount of light (e.g., 1461, 1463, 1465, and 1467 in FIG. 14) absorbed and reflected by the light irradiation area 1020 (e.g., skin of a part of the user's body (e.g., the wrist)) is defined as R_{1,1}. In this case, the processor 120 may create a first-first point 1541 on the basis of R_{1,1}/M_{1,1}.

As another example, in case that the light in a first-second wavelength range 1520 (e.g., λ_{1,2}) is emitted from the first-second light source 523 of the first light-emitting structure 520, the monitoring circuits (e.g., the first monitoring circuit 1410 and the second monitoring circuit 1415 in FIG. 14) may acquire (or receive) at least a part of the light reflected or scattered by the rear surface plate 1010. In addition, in case that the light in the first-second wavelength range 1520 is emitted from the first-second light source 523 of the first light-emitting structure 520, the light detection circuits (e.g., the first light detection circuit 1430 and the second light detection circuit 1435 in FIG. 14) may acquire (or receive) the light absorbed and reflected by the light irradiation area 1020 (e.g., skin of a part of the user's body (e.g., the wrist)). The description will be made on the assumption that the amount of acquired (or received) light reflected or scattered by the rear surface plate 1010 is defined as M_{1,2}, and the amount of light absorbed and reflected by the light irradiation area 1020 (e.g., skin of a part of the user's body (e.g., the wrist)) is defined as R_{1,2}. In this case, the processor 120 may create a first-second point 1543 on the basis of R_{1,2}/M_{1,2}.

In case that the light in an n-th-first wavelength range 1525 (e.g., λ_{n,1}) to an n-th-eighth wavelength range 1530 (e.g., λ_{n,8}) is emitted from the n-th-first light source to the n-th-eighth light source of the n-th light-emitting structure 570 in the above-mentioned way, the monitoring circuits (e.g., the first monitoring circuit 1410 and the second monitoring circuit 1415 in FIG. 14) may acquire (or receive) at least a part of the light reflected or scattered by the rear surface plate 1010. In addition, in case that the light in the first-second wavelength range is emitted from the first-second light source 523 of the first light-emitting structure 520, the light detection circuits (e.g., the first light detection circuit 1430 and the second light detection circuit 1435 in FIG. 14) may acquire (or receive) the light absorbed and reflected by the light irradiation area 1020 (e.g., skin of a part of the user's body (e.g., the wrist)). The processor 120 may create an n-th-first point 1545 to an n-th-eighth point 1547 on the basis of the amount of acquired (or received) light reflected or scattered by the rear surface plate 1010 and the amount of light absorbed and reflected by the light irradiation area 1020 (e.g., skin of a part of the user's body (e.g., the wrist)).

In the embodiment, it is possible to generate a spectrum for each wavelength range by connecting (or correcting) the first-first point 1541, the first-second point 1543, ..., and the n-th-first point 1545 to the n-th-eighth point 1547. The processor 120 may measure at least one bio-information by analyzing the generated spectrum for each wavelength range.

The electronic device 101 or 200 according to the embodiment of the disclosure may include the housing 210 including the first surface 210A, the second surface 210B opposite to the first surface 210A, and the side surface 210C configured to surround the first surface 210A and the second surface 210B. The electronic device 101 or 200 according to the embodiment may include the substrate 601 disposed in the housing 210. The electronic device 101 or 200 according to the embodiment may include at least one of the light-emitting structures 520, 540, and 570 disposed on the substrate 601 so as to be directed toward the second surface 210B of the housing 210 and formed in the form of a bar. The electronic device 101 or 200 according to the embodiment may include the plurality of light sources 5200 and 5700 configured to emit light in different wavelength ranges and disposed at designated intervals on at least one of the light-emitting structures 520, 540, and 570 formed in the form of a bar. At least one of the light-emitting structures 520, 540, and 570 according to the embodiment may be electrically connected to the substrate 601 by means of soldering in the state in which at least one of the light-emitting structures 520, 540, and 570 is inclined at a particular angle.

The particular angle according to the embodiment may be configured based on a distance between each of the light-emitting structures 520, 540, and 570 and the light irradiation area 1020 so that the light emitted from the plurality of light sources 5200 and 5700 reaches the light irradiation area 1020.

The particular angle according to the embodiment may be configured further based on the configuration in which the light is reflected by the second surface 210B between each of the light-emitting structures 520, 540, and 570 and the light irradiation area 1020.

The plurality of light sources 5200 and 5700 included in at least one of the light-emitting structures 520, 540, and 570 according to the embodiment may emit light in different wavelength ranges through different optical paths based on a configuration in which at least one of the light-emitting structures 520, 540, and 570 is electrically connected to the substrate 601 in the state in which at least one of the light-emitting structures 520, 540, and 570 is inclined at the particular angle.

At least one of the light-emitting structures 520, 540, and 570 according to the embodiment may be electrically connected to the substrate 601 by means of the soldering members 615, 625, or 635, each of which is disposed on at least a part of the first surface 5201, 5401, or 5701 of at least one of the light-emitting structures 520, 540, and 570, and the soldering members 620, 630, or 640 disposed on at least a part of the second surface 5202, 5402, or 5702 of at least one of the light-emitting structures 520, 540, and 570.

The soldering members 615, 625, or 635 disposed on at least a part of the first surface 5201, 5401, or 5701 of at least one of the light-emitting structures 520, 540, and 570 according to the embodiment may include the plurality of first soldering members 6151, 6152, 6153, 6154, 6155, 6156, 6157, and 6158.

The plurality of first soldering members 6151, 6152, 6153, 6154, 6155, 6156, 6157, and 6158 according to the embodiment may be disposed on the first surface 5201, 5401, or 5701 of at least one of the light-emitting structures 520, 540, and 570 so as to overlap at least a part of each of the light sources.

The electronic device 101 or 200 according to the embodiment may further include at least one of the light absorption structures 820, 830, and 840 disposed on the substrate 601. At least one of the light-emitting structures 520, 540, and 570 according to the embodiment may be disposed on at least one of the light absorption structures 820, 830, and 840.

In the embodiment, when at least a part of the light emitted from the plurality of light sources 5200 and 5700 included in at least one of the light-emitting structures 520, 540, and 570 is transmitted to the substrate 601, at least a part of the light transmitted to the substrate 601 may be absorbed by at least one of the light absorption structures 820, 830, and 840.

The electronic device 101 or 200 according to the embodiment may further include at least one of the insulators 920, 930, and 940 disposed on at least a part of the first surface 5201, 5401, or 5701 of at least one of the light-emitting structures 520, 540, and 570.

At least one of the insulators 920, 930, and 940 according to the embodiment may be disposed on the first surface 5201, 5401, or 5701 of at least one of the light-emitting structures 520, 540, and 570 and disposed between the plurality of light sources 5200 and 5700 included in at least one of the light-emitting structures 520, 540, and 570.

The soldering members 615, 625, or 635 according to the embodiment may be disposed on at least a part of the first surface 5201, 5401, or 5701 of at least one of the light-emitting structures 520, 540, and 570 after at least one of the insulators 920, 930, and 940 is disposed on at least a part of the first surface 5201, 5401, or 5701 of at least one of the light-emitting structures 520, 540, and 570.

The electronic device 101 or 200 according to the embodiment may further include the driver 1405 and the processor 120. The processor 120 according to the embodiment may control the driver 1405 so that the plurality of light sources 5200 and 5700 sequentially emits light.

The electronic device 101 or 200 according to the embodiment may further include at least one of the monitoring circuits 1111, 1151, and 1171 disposed on the substrate 601.

At least one of the monitoring circuits 1111, 1151, and 1171 according to the embodiment may identify an intensity and wavelength range of the light emitted from the plurality of light sources 5200 and 5700. At least one of the monitoring circuits 1111, 1151, and 1171 according to the embodiment may be transmitted to identified intensity and wavelength range of the light to the processor 120.

The electronic device 101 or 200 according to the embodiment may further include the light detection circuit disposed on the substrate 601.

The processor 120 according to the embodiment may detect the light, which is emitted from the plurality of light sources 5200 and 5700 and reflected by the light irradiation area 1020, by means of the light detection circuit. The processor 120 according to the embodiment may acquire at least one bio-information on the basis of the light reflected by the light irradiation area 1020 and detected by the light detection circuit.

The plurality of light sources 5200 and 5700 according to the embodiment may include laser diodes.

Instead of the soldering members 615, 625, or 635, the wires 720, 730, or 740 may be disposed on the first surface 5201, 5401, or 5701 of at least one of the light-emitting structures 520, 540, and 570 according to the embodiment.

At least one of wires 720, 730, or 740 according to the embodiment may include the plurality of wires 7201, 7202, 7203, 7204, 7205, 7206, 7207, and 7208. The plurality of wires 7201, 7202, 7203, 7204, 7205, 7206, 7207, and 7208 according to the embodiment may each be connected to at least a part of each of the plurality of light sources 5200 and 5700.

It will be appreciated that various embodiments of the disclosure according to the claims and description in the specification can be realized in the form of hardware, software or a combination of hardware and software.

Any such software may be stored in non-transitory computer readable storage media. The non-transitory computer readable storage media store one or more computer programs (software modules), the one or more computer programs include computer-executable instructions that, when executed by one or more processors of an electronic device, cause the electronic device to perform a method of the disclosure.

Any such software may be stored in the form of volatile or non-volatile storage such as, for example, a storage device like read only memory (ROM), whether erasable or rewritable or not, or in the form of memory such as, for example, random access memory (RAM), memory chips, device or integrated circuits or on an optically or magnetically readable medium such as, for example, a compact disk (CD), digital versatile disc (DVD), magnetic disk or magnetic tape or the like. It will be appreciated that the storage devices and storage media are various embodiments of non-transitory machine-readable storage that are suitable for storing a computer program or computer programs comprising instructions that, when executed, implement various embodiments of the disclosure. Accordingly, various embodiments provide a program comprising code for implementing apparatus or a method as claimed in any one of the claims of this specification and a non-transitory machine-readable storage storing such a program.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively," as "coupled with," "coupled to," "connected with," or "connected to" another element (e.g., a second element), it means that the element may be coupled with the other element directly (e.g., through wires), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry." A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. The term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

While the disclosure has been shown and described with reference to various embodiments thereof, it will be understood by those skilled in the art that various changes in form and details may be made therein without departing from the spirit and scope of the disclosure as defined by the appended claims and their equivalents.

## Claims

1. An electronic device (101, 200) comprising:
a housing (210) including a first surface (210A), a second surface (210B) opposite to the first surface (210A), and a side surface (210C) surrounding the first surface (210A) and the second surface (210B);
a substrate (601) disposed in the housing (210);
at least one light-emitting structure (520, 540, 570) being disposed on the substrate (601) so as to be directed toward the second surface (210B) and being formed in a form of a bar; and
a plurality of light sources (5200, 5700) configured to emit light in different wavelength ranges and being disposed at designated intervals on the at least one light-emitting structure (520, 540, 570) formed in the form of the bar,
wherein the at least one light-emitting structure (520, 540, 570) is electrically connected to the substrate (601) by soldering in a state in which at least one of the at least one light-emitting structure (520, 540, 570) is inclined at a particular angle.

2. The electronic device of claim 1, wherein the particular angle is configured based on a distance between each of the at least one light-emitting (520, 540, 570) structure and a light irradiation area (1020) so that the light emitted from the plurality of light sources (5200, 5700) reaches the light irradiation area (1020).

3. The electronic device of claim 2, wherein the particular angle is further configured based on a configuration in which the light is reflected by a second surface (210B) between each of the at least one light-emitting structure (520, 540, 570) and the light irradiation area (1020).

4. The electronic device of any of claims 1 to 3, wherein the plurality of light sources (5200, 5700) included in the at least one light-emitting structure (520, 540, 570) emits the light in the different wavelength ranges through different optical paths based on a configuration in which the at least one light-emitting structure (520, 540, 570) is electrically connected to the substrate (601) in a state in which the at least one light-emitting structure (520, 540, 570) is inclined at the particular angle.

5. The electronic device of any of claims 1 to 4, wherein the at least one light-emitting structure (520, 540, 570) is electrically connected to the substrate (601) by a first soldering member (615, 625, 635), which is disposed on at least a part of a first surface (5201, 5401, 5701) of the at least one light-emitting structure (520, 540, 570), and a second soldering member (620, 630, 640) disposed on at least a part of a second surface (5202, 5402, 5702) of the at least one light-emitting structure (520, 540, 570).

6. The electronic device of claim 5, wherein the first soldering member (615, 625, 635) disposed on at least the part of the first surface (5201, 5401, 5701) of the at least one light-emitting structure (520, 540, 570) comprises a plurality of first soldering members (6151, 6152, 6153, 6154, 6155, 6156, 6157, 6158), and
wherein the plurality of first soldering members are disposed on the first surface of the at least one light-emitting structure so as to overlap at least a part of each of the plurality of light sources.

7. The electronic device of any of claims 1 to 6, further comprising:
at least one light absorption structure (820, 830, 840) disposed on the substrate,
wherein the at least one light-emitting structure (520, 540, 570) is disposed on the at least one light absorption structure (820, 830, 840).

8. The electronic device of claim 7, wherein, in response to at least a part of the light emitted from the plurality of light sources (5200, 5700) included in the at least one light-emitting structure (520, 540, 570) is transmitted to the substrate (601), at least a part of the light transmitted to the substrate (601) is absorbed by the at least one light absorption structure (820, 830, 840).

9. The electronic device of any of claims 1 to 8, further comprising:
at least one insulator (920, 930, 940) disposed on at least a part of a first surface (5201, 5401, 5701) of the at least one light-emitting structure (520, 540, 570),
wherein the at least one insulator (920, 930, 940) is disposed on the first surface (5201, 5401, 5701) of the at least one light-emitting structure (520, 540, 570) between the plurality of light sources (5200, 5700) included in the at least one light-emitting structure (520, 540, 570).

10. The electronic device of claim 8 or 9, wherein a first soldering member (615, 625, 635) is disposed on at least a part of the first surface (5201, 5401, 5701) of the at least one light-emitting structure (520, 540, 570) after the at least one insulator (920, 930, 940) is disposed on at least the part of the first surface (5201, 5401, 5701) of the at least one light-emitting structure (520, 540, 570).

11. The electronic device of any of claims 1 to 10, further comprising:
a driver (1405); and
one or more processors (120),
wherein the one or more processors (120) is configured to control the driver (1405) so that the plurality of light sources (5200, 5700) sequentially emit the light.

12. The electronic device of any of claims 1 to 11, further comprising:
at least one monitoring circuit (1111, 1151, 1171) disposed on the substrate (601),
wherein the at least one monitoring circuit is configured to:
identify an intensity and wavelength range of the light emitted from the plurality of light sources; and
transmit the identified intensity and wavelength range of the light to the one or more processors.

13. The electronic device of any of claims 1 to 12, further comprising:
a light detection circuit disposed on the substrate (601),
wherein the one or more processors (120) is further configured to:
detect, through the light detection circuit, the light emitted from the plurality of light sources (5200, 5700) and reflected by the light irradiation area; and
acquire at least one piece of biometric information based on the light reflected by the light irradiation area (1020) detected through the light detection circuit.

14. The electronic device of any of claims 1 to 13, wherein the plurality of light sources (5200, 5700) comprises laser diodes.

15. The electronic device of claim 3, wherein a wire (720, 730, 740) is disposed on a first surface (5201, 5401, 5701) of the at least one light-emitting structure (520, 540, 570),
wherein the wire (720, 730, 740) comprises a plurality of wires (7201, 7202, 7203, 7204, 7205, 7206, 7207, 7208), and the plurality of wires (7201, 7202, 7203, 7204, 7205, 7206, 7207, 7208) is each connected to at least a part of each of the plurality of light sources (5200, 5700).
